# EUROPEAN PATENT SPECIFICATION

(11) **EP 2 064 385 B1**
(45) Date of publication and mention of the grant of the patent: **17.02.2016**
(21) Application number: 07838591.1
(22) Date of filing: 20.09.2007
(51) Int. Cl.: D06M 16/00, D06L 1/14, D06L 3/02, D06L 3/06, D06L 3/11, C11D 3/386, C12N 9/88, D06P 1/00

(54) **ENZYMATIC TREATMENT OF TEXTILES USING A PECTATE LYASE FROM BACILLUS SUBTILIS**
ENZYMATISCHE BEHANDLUNG VON TEXTILSTOFFEN MITHILFE EINER PECTAT-LYASE VON BACILLUS SUBTILIS
TRAITEMENT ENZYMATIQUE DE TEXTILES UTILISANT UNE PECTATE LYASE OBTENUE DU BACILLUS SUBTILIS

(30) Priority: 22.09.2006 US 846329 P
(43) Date of publication of application: 03.06.2009
(73) Proprietor: Danisco US, Inc., Genencor Division, Palo Alto, CA 94304 (US)
(72) Inventor: POULOSE, Ayrookaran J., Palo Alto, CA 9430 (US); SCHMIDT, Brian, Palo Alto, CA 94304 (US); VAN GASTEL, Franciscus J.C., Palo Alto, CA 94304 (US); YOON, Mee-young, Palo Alto, CA 94304 (US)
(74) Representative: Kremer, Simon Mark
(86) International application number: PCT/US2007/020410
(87) International publication number: WO 2008/039353

(56) References cited:
- WO-A-00/71808
- WO-A-02/092741
- WO-A-03/002810
- WO-A-03/095638
- NASSER W ET AL: "PECTATE LYASE FROM BACILLUS SUBTILIS: MOLECULAR CHARACTERIZATION OFTHE GENE, AND PROPERTIES OF THE CLONED ENZYME" FEBS LETTERS, ELSEVIER, AMSTERDAM, NL, vol. 335, no. 3, 1 January 1993 (1993-01-01), pages 319-326, XP000918458 ISSN: 0014-5793

## Description

### FIELD OF THE INVENTION

Compounds and compositions comprising a pectate lyase of *Bacillus subtilis,* its use in bioscouring, bleaching, dyeing, desizing, cellulosic fiber treatment, and cleaning compositions, and combinations thereof are described herein.

### BACKGROUND OF THE INVENTION

Pectin polymers are constituents of plant cell walls. Pectin is a hetero-polysaccharide with a backbone comprised of alternating homogalacturonan (smooth regions) and rhamnogalacturonan (hairy regions). Linear polymers of 1,4-linked α-D-galacturonic acid forms the smooth regions. The galacturonic acid moieties can be methyl-esterified on the carboxyl group to a varying degree, usually in a non-random fashion with blocks of polygalacturonic acid being completely methyl-esterified.

Pectinases can be classified by preferential substrate, *i.e.* highly methyl-esterified pectin or low methyl-esterified pectin and polygalacturonic acid (pectate). They can also be classified by their reaction mechanism, *i.e.* beta-elimination or hydrolysis. Pectinases can be mainly endo-acting, *i.e.* cutting the polymer at random sites within the chain to give a mixture of oligomers. Alternatively, pectinases can be exo-acting, whereby they attack from one end of the pectin. Several pectinase activities that act on the smooth regions of pectin are included in the classification of enzymes provided by the Enzyme Nomenclature (1992) such as pectate lyase (EC 4.2.2.2), pectin lyase (EC 4.2.2.10), polygalacturonase (EC 3.2.1.15), pectin esterase (EC 3.2.1.11), galactanase (EC 3.2.1.89), arabinase (EC 3.2.1.99) and mannanase (EC 3.2.1.78).

Pectate lyases have been cloned from different bacterial genera. To date, most of the pectate lyases that have been purified have a pH optimal activity of eight or greater, typically have an optimal temperature range of 50°C or greater, and require divalent cations for maximum activity, with calcium ions being the most stimulatory.

Compositions capable of desizing, bleaching, scouring, dyeing and one-step combinations thereof are needed, as are cleaning reagents. In the textile processing of natural fibers, yarns and fabrics, a pretreatment or preparation step is typically required to properly prepare the natural materials for further use and in particular for the dyeing and/or finishing stages typically required for commercial goods. These textile treatment steps remove impurities and color bodies, either naturally existing or those added by the spinning and weaving steps to the fibers and/or fabrics.

While textile treatments may include a number of varying treatments and stages, the most common include: de-sizing - the removal of sizing agents, such as starches, via enzymatic, alkali or oxidative soaking; scouring - the removal of greases, oils, waxes, pectic substances, motes, protein and fats by contact with a solution of sodium hydroxide at temperatures near boiling; and bleaching - the removal and lightening of color bodies from textiles by commonly using oxidizing agents (such as hydrogen peroxide, hyphochlorite, and chlorine dioxide), or by using reducing agents (such as, sulfur dioxide or hydrosulfite salts).

Commercial enzymatic textile processing typically requires the separation of these pretreatment steps due to the broad variation of conditions present in each of the steps. However, this separation of treatment steps leads to heavy additional costs added to the overall treatment process due to the requirement of multiple rinsing steps required between the respective stages, and high energy costs due to high processing temperature above 95°C. The additional rinse and/or drying steps add enormous additional costs, time, and waste materials to the treatment process.

Accordingly, the combination of various pre-treatment stages into a one-step treatment would have a significant environmental benefit and significant impact in the commercial treatment of textiles in the form of reduced costs and waste materials over the commercial processes typically employed.

However, the combination of these three common steps, while previously investigated, has been unsatisfactory. Currently employed bleaching technology involves the use of alkaline hydrogen peroxide bleaching at temperatures in excess of 95° C. Such high temperatures and strong bleaching systems are wholly incompatible with the many enzymes utilized in a de-sizing operation. Thus, the combination of the de-sizing and bleaching technology at temperatures in excess of 95°C leads to destruction of the de-sizing enzymes and an unsatisfactory de-sizing result. Alternative de-sizing techniques, such as alkali or oxidative soaking, involves the use of aggressive chemicals which lead to fiber damage. On the other hand, reduction of the temperature at which the one-step treatment is conducted to allow effective enzymatic de-sizing results in an unacceptably poor bleaching with whiteness values below the commercially acceptable limit. Furthermore, this kind of low temperature process without a scouring enzyme produces a fabric of low wettability that is unacceptable for further dyeing, printing and finishing processes.

Cleaning stains involves the removal of colored substances that comprise the stains. A common type of stain originates from vegetable materials, such as stains from grass, vegetables (spinach, beets, carrots, tomatoes, fruits such as berries, and grapes), and plant derived materials such as wine, beer, fruit juices, tomato sauces and the like. The pigments usually adhere to fibrous materials via covalent bonds or via physical binding ("sticking"). Removal of these pigments can be very difficult, since detergents can barely remove the fiber-pigment mass from a surface needing cleaning. Research has shown that plant cell walls consist of a complicated network of fibrous materials. The composition of the cell walls varies considerably depending on the source of the vegetable material. However, in general, a plant's composition comprises non-starch polysaccharides. These polysaccharides exist in various forms, including for example cellulose, hemicellulose, and pectins.

Thus, there is a continued need to identify pectate lyases for use in bioscouring, bleaching, desizing, dyeing steps and combinations thereof, as well as in detergent compositions, cleaning compositions, which have an optimal temperature between 10-50°C *(e.g.,* 20-30°C), operates optimally in an acidic to slightly basic environment (pH < 8:5), and is not dependent on the presence of divalent cations, especially calcium.

### SUMMARY OF THE INVENTION

The invention provides a method for scouring and bleaching a textile as defined in the claims.

Accordingly, described herein are compounds, compositions and methods of using same to bioscour, bleach, desize, and/or dye cellulosic fibers, as well as clean hard substrates and textiles. The methods and compositions described herein obviates one or more of the problems due to limitations and disadvantage that are currently experienced in the art.

Thus, one aspect described herein is a pectate lyase comprising the polypeptide of SEQ ID NO: 1. Further described are pectate lyases obtained from *Bacillus subtilis* having a molecular weight of about 43 kD, and a pI of about 7.3 under reducing SDS-PAGE conditions. An optimal pH for raw cotton is about 6.0 to 8.0, however would vary if another substrate is used.

Also described herein is a nucleic acid encoding SEQ ID NO: 1, such as SEQ ID NO: 2. Vectors comprising said nucleic acid operably linked to the vector are contemplated as are host cells comprising the vector and expressing the polypeptide encoded by the nucleic acid. Host cells can be any prokaryotic or eukaryotic host cells, such as species of *Bacillus.*

A further aspect described herein is a bioscouring composition comprising one of the described pectate lyases in an aqueous solution. The bioscouring composition can further comprise a bleaching agent or bleaching composition. Bleaching agent include but are not limited to an oxidative bleach, sodium peroxide, sodium hypochlorite, calcium hypochlorite, sodium dichloroisocyanurate, or a combination thereof. The bleaching composition can be for example an ester source, an acyl transferase, and a hydrogen peroxide source. The bioscouring composition and/or bioscouring and bleaching composition can further comprise a desizing agent. The bioscouring compositions can further comprise additional bioscouring enzymes such as a pectinase, a cutinase, a cellulase, a hemicellulase, a protease, a lipase, or a combination thereof.

Also described herein is a one-step treatment composition for textiles is contemplated. The composition comprises: (a) a pectate lyase obtained from *Bacillus subtilis* having a molecular weight of about 43 kD, a pI of about 7.3 under reducing SDS-PAGE conditions; (b) one or more desizing enzymes; and (c) one or more bleaching agents and/or bleaching composition. It would be apparent that bleaching and dyeing would not occur in the same step, but can be combined with scouring and desizing. The composition can also further comprise a bioscouring enzyme selected from the group consisting of a pectinase, a cutinase, a cellulase, a hemicellulase, a protease, a lipase, and a combination thereof. The one-step treatment composition can further comprise one or more auxiliary components, wherein said auxiliary component is a surfactant, an emulsifier, a chelating agent, and/or a stabilizer, or a combination thereof. The composition can further comprise a bleach activator. The bleaching agents for use in said one-step treatment compositions include but are not limited to an oxidative bleach, sodium peroxide, sodium hypochlorite, calcium hypochlorite, sodium dichloroisocyanurate, or a combination thereof. The surfactants for use in said one-step treatment composition can include but are not limited to a non-ionic surfactant, an anionic surfactant, a cationic surfactant, a zwitterionic surfactant, or a combination thereof.

The method for scouring and bleaching a textile comprises contacting the textile for a length of time and under conditions suitable to permit whitening of the textile, with an aqueous solution comprising a bleach and a pectate lyase obtained from *Bacillus subtilis* having a molecular weight of about 43 kD, a pI of about 7.3 under reducing SDS-PAGE conditions. Suitable conditions that permit whitening include a pH of about 5 to about 11; a time between about 2 minutes and 24 hours, more preferably between about 15 minutes and 12 hours; and more preferably between about 30 minutes and 6 hours; a temperature of between about 15°C and 90°C. Suitable temperature can be decided based on the process used for the treatment. Suitable temperature for cold pad batch scouring and bleaching is about 15°C to about 45°C and preferably about 25°C to about 35°C. Temperatures for continuous, semi-continuous and other batch processes include about 15°C to about 90°C, preferably about 20°C to about 75°C, and more preferably about 25°C to about 60°C. The bleaching agent can be hydrogen peroxide and present in an amount of between about 100 ppm to 5000 ppm. Thus one exemplary set of conditions contemplated comprises a pH of about 6 to 8, a length of time of about 2 minutes to 24 hours, a temperature of about 25°C to 60°C, and wherein the bleach is hydrogen peroxide and is present in an amount of about 1000 ppm to 3000 ppm.

Also described herein is a method for one-step processing of textiles comprising: (a) providing a one-step textile processing composition and a textile in need of processing, and wherein said one-step textile processing composition comprises a pectate lyase obtained from *Bacillus subtilis* having a molecular weight of about 43 kD, a pI of about 7.3 under reducing SDS-PAGE conditions; (b) contacting said textile with said one-step textile processing composition for a length of time and under conditions sufficient to permit desizing, scouring, and bleaching of the textile. The one-step textile processing composition can further comprise: (a) one or more bioscouring enzymes; (b) one or more bleaching agents and/or bleaching compositions; and (c) one or more desizing agents. One or more bioscouring enzymes can include but are not limited to a pectinase, a cutinase, a protease, a cellulase, a hemicellulase, a lipase, or a combination thereof. Desizing agents can include but are not limited to an amylase, a cellulase, a mannanase, or a combination thereof. The one-step textile processing composition can further comprises an auxiliary component selected from the group consisting of a surfactant, an emulsifier, a chelating agent, a stabilizer, or a combination thereof. Bleaching agents for use in the contemplated one-step textile processing composition included but are not limited to an enzymatic bleaching systems, and chemical bleaches such as but not limited to oxidative bleach, sodium peroxide, sodium hypochlorite, calcium hypochlorite, sodium dichloroisocyanurate, or combinations thereof. The textiles for treatment are those comprising a cellulosic or cellulosic-containing material, *e.g.*, cotton. The conditions for performing the one-step processing can be performed at a temperature of about 15°C and 90°C, preferably from about 20°C to about 75°C, and more preferably from about 25°C to about 60°C, and a pH of about 5 to 11, more preferably from about 6 to about 10, and more preferably from about a pH of about 6 to about 8, for a time between about 2 minutes and 24 hours. The suitable treatment temperature and pH can be decided depending on the process selected from the treatment.

Also described herein is a method for textile preparation that comprises simultaneous or sequential bioscouring and bleaching, wherein said scouring step comprises contacting said textile an aqueous composition comprising a *Bacillus subtilis* pectate lyase having a molecular weight of about 43 kD, a pI of about 7.3 under reducing SDS-PAGE conditions, and wherein said contacting occurs under conditions suitable for bioscouring.

Also described herein is a method of bioscouring and dyeing a textile comprising: (a) preparing an aqueous solution comprising a *B. subtilis* pectate lyase having a molecular weight of about 43 kD, a pI of about 7.3 under reducing SDS-PAGE conditions, and an optimal pH of about 6.0 to 8.0, and wherein said pectate lyase is present in an amount of about 10 to 500 mol/min/kg of textile at a pH of about 5 to 11, preferably a pH of about 5 to 9, and more preferably a pH from about 6 to about 8, and a temperature of about 15°C to 90°C (or from about 20°C to about 75°C, or more preferably from about 25°C to about 60°C) and having less than 2 mM divalent cations; (b) incubating the textile in the aqueous solution for a time sufficient for bioscouring; and (c) supplementing the aqueous solution with a dyeing system and incubating the textile for a sufficient time to achieve dyed textile.

Also described herein is a detergent composition comprising: (a) a surfactant; (b) a *B. subtilis* pectate lyase having a molecular weight of about 43 kD, a pI of about 7.3 under reducing SDS-PAGE conditions; and (c) optionally a detergent additive wherein said detergent candidate is a builder, a bleaching agent, an antifoaming agent, an abrasive, a suspending agent, a soil-releasing agent, a bactericide, a lubricants, or a combination thereof. The contemplated detergent composition can further comprise a hemicellulase enzyme such as, but not limited to, xylanase, arabinofuranosidase, acetyl xylan esterase, glucuronidase, ferulic acid esterase, courmaric acid esterase, endo-galactanase, mannanase, lichenase, endo-arabinase, exo-arabinase, exo-galactanase, or a combination thereof. The detergent composition can further comprise a protease, a cellulase, a lipase, or a combination thereof.

Also described herein is a method for cleaning a textile comprising exposing a textile in need of cleaning with a detergent composition comprising: (a) a surfactant; (b) a *B. subtilis* pectate lyase having a molecular weight of about 43 kD, a pI of about 7.3 under reducing SDS-PAGE conditions; and (c) optionally a detergent additive wherein said detergent candidate is a builder, a bleaching agent, an antifoaming agent, an abrasive, a suspending agent, a soil-releasing agent, a bactericide, a lubricants, or a combination thereof. The detergent composition can further comprise a protease, a cellulase, a lipase, or a combination thereof.

Also described herein are compositions and methods for cleaning hard surfaces. Said composition can comprise an aqueous solution comprising a *B*. *subtilis* pectate lyase having a molecular weight of about 43 kD, a pI of about 7.3 under reducing SDS-PAGE conditions, and an optimal pH of about 6.0 to 8.0. The range can be larger for cleaning and detergent compositions, *e*.*g*., from about a pH of 5 to 11.

It is to be understood that both the foregoing general description and the following detailed description are exemplary and explanatory and are intended to provide further explanation of the compounds, compositions, and methods disclosed.

### BRIEF DESCRIPTION OF THE DRAWING

The accompanying drawings are incorporated in and constitute a part of this specification, and illustrate. Together with the description, the figures serve to illustrate the aspects of the compositions and methods discussed.

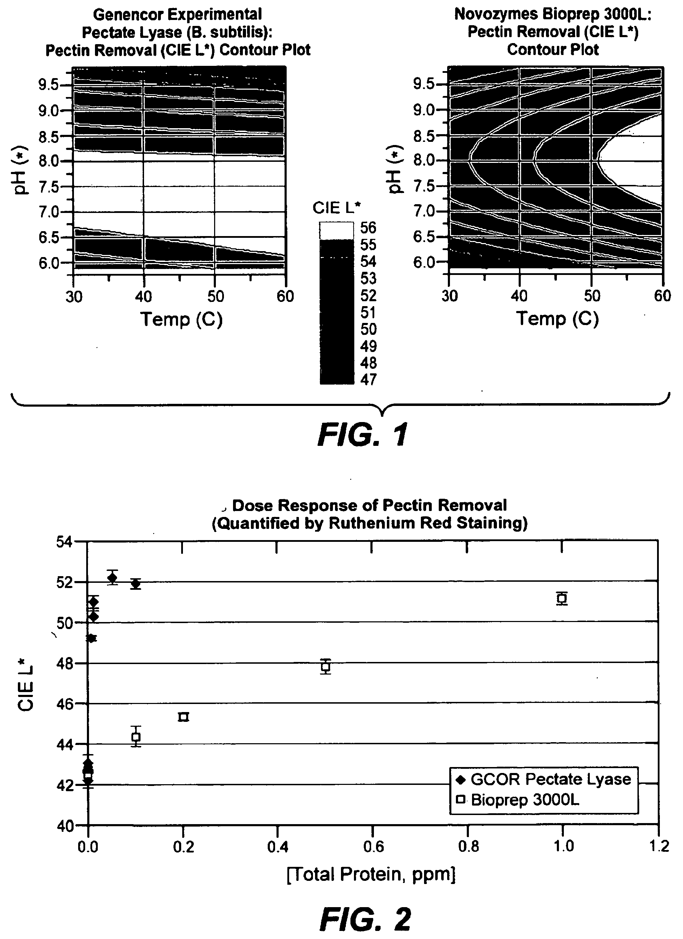

**FIG. 1** depicts the pH range of *B. subtilis* pectate lyase Exp. *B. subtilis* on pectin removal with pH on the y-axis and temperature (°C) on the x-axis. The *B. subtilis* pectate lyase (left panel) was compared to the commercially available pectate lyase activity of Bioprep 3000L (right panel). 2 ppm each of enzyme was incubated with unscoured cotton (Testfabrics, 428U) for 1 hour under different pH (5.9-9.8) and temperature (30-60°C) conditions to compare preliminary pH and temperature profiles of the *B. subtilis* pectate lyase and Bioprep 3000L. The experimental design and data analysis were done using a statistical software program. After enzyme treatment and a thorough rinsing of the cotton fabric, the treated fabric disks were stained with Ruthenium red dye to quantify the amount of pectin remaining in the fabric after the treatments. The stained fabrics were then thoroughly rinsed and air-dried before measuring the CIE L* values using a Minolta CR-200 spectrophotometer. The lower CIE L* indicates higher pectin binding. The higher the pectin binding, the lower the scouring performance of the enzyme. In the figure, the darker shading corresponds to less pectin removal.
**FIG. 2** depicts the dose response of pectin removal from raw cotton fabric. The experiment was performed at 55°C for 20 minutes at a pH of 7.5 for the *B. subtilis* pectate lyase (GCOR). For Bioprep 3000L, the assay was again performed at 55°C for 20 minutes, but the pH was increased to 8.2 to test the enzyme under its optimal pH. The x-axis is presented in total protein concentration (ppm). The y-axis is in CIE L* units. CIE is the Commission Internationale de l'Eclairage. L is the light intensity parameter. "a" is a the green to red parameter and "b" is the blue to yellow parameter.
**FIG. 3** depicts pectin removal performance of *B. subtilis* pectate lyase at 0.1 ppm (left panel), 0.5 ppm (middle panel), and 1 ppm (right panel) across pH (y-axis) and temperature (°C) (x-axis). The *B. subtilis* pectate lyase had a broad temperature spectrum *(i.e.,* 25-65°C) and broad pH spectrum.

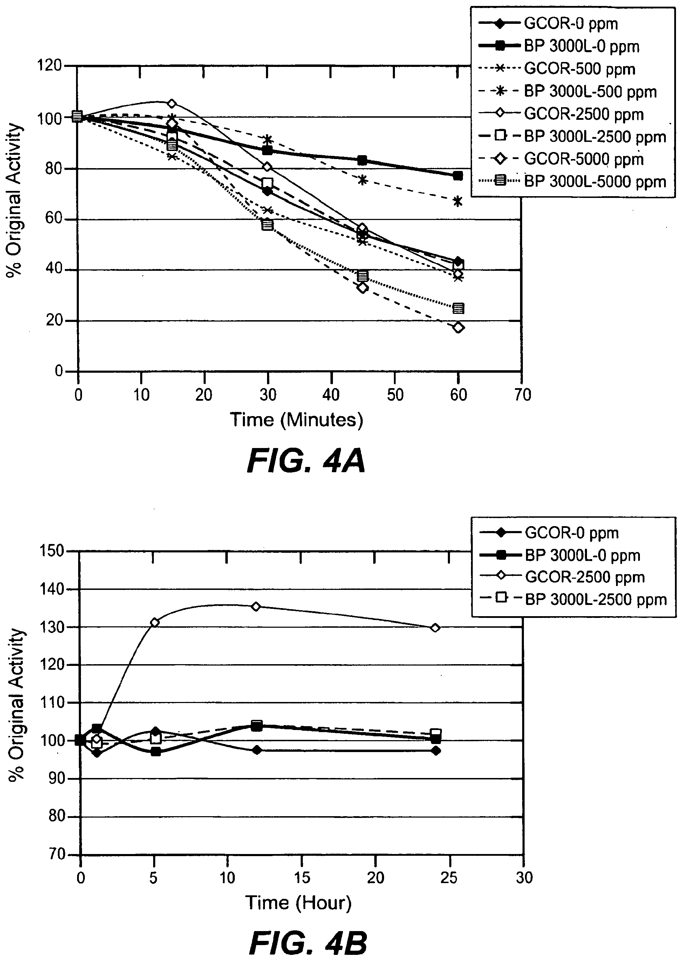

**FIG. 4** depicts the stability of *B. subtilis* pectate lyase as compared to a commercially available pectate lyase at different temperatures and with different concentrations of hydrogen peroxide. FIG. 4A shows the stability of the enzyme at 55°C. 0, 500, 2500 and 5000 ppm of hydrogen peroxide and compares *B. subtilis* pectate lyase (GCOR) and Bioprep 3000L. FIG. 4B depicts the results obtained at 0 ppm and 2500 ppm of hydrogen peroxide as tested at 25°C in the same amount of pectate lyase over time. The activity of the *B. subtilis* pectate lyase (GCOR) actually increased over time at 25°C.
**FIG. 5** depicts pectate lyase stability in the presence or absence of the chelator, EDTA, and at room temperature (RT) or at 55°C. Activity was measured as the percent (%) activity lost at 1 hour. The activity of the *B. subtilis* pectate lyase (GCOR) was compared to BioPrep 3000L.
**FIG. 6****.** NuPAGE 4-12% Bis-Tris Gel with MES running buffer demonstrating the purity of the *B. subtilis* pectate lyase and its molecular weight relative to controls.
**FIGS. 7A** **and** **7B****.** Panels A and B show cleaning by 10ppm *B. subtilis* pectate lyase in 0.15mL/L AATCC WOB 2003 heavy-duty liquid detergent on fresh stains.
**FIGS. 8A** **and** **8B****.** Panels A and B show cleaning by 1ppm *B. subtilis* pectate lyase in 1.5mL/L AATCC WOB 2003 heavy-duty liquid detergent on fresh stains.
**FIG. 9****.** depicts the comparision of cleaning performance of *B. subtilis* pectate lyase compared with *B. licheniformis* amylase in standard AATCC WOB 2003 heavy-duty liquid detergent on fresh stains.
**FIG. 10****.** depicts the cleaning performance of *B. subtilis* pectate lyase compared with *B. licheniformis* amylase in a standard AATCC WOB liquid detergent on aged technical stains.
**FIG. 11****.** depicts the cleaning performance of B. subtilis pectate lyase compared with Bacillus sp. 707 amylase in a commercial liquid detergent on fresh stains
**FIG.12****.** depicts the cleaning performance of B. subtilis pectate lyase compared with Bacillus sp. 707 amylase in a commercial liquid detergent on aged technical stains.

### DETAILED DESCRIPTION OF THE PREFERRED EMBODIMENTS

The compositions, compounds, and methods will now be described in detail by way of reference only using the following definitions and examples.

Numeric ranges are inclusive of the numbers defining the range.

Unless otherwise indicated, nucleic acids are written left to right in 5' to 3' orientation; amino acid sequences are written left to right in amino to carboxy orientation, respectively.

The headings provided herein are not limitations of the various aspects or embodiments of the disclosed compounds, compositions, and methods, which can be had by reference to the specification as a whole. Accordingly, the terms defined immediately below are more fully defined by reference to the specification as a whole.

### 1. Abbreviation and Definitions

### 1.1 Definitions

Unless defined otherwise herein, all technical and scientific terms used herein have the same meaning as commonly understood by one of ordinary skill in the art. For example, Singleton and Sainsbury, DICTIONARY OF MICROBIOLOGY AND MOLECULAR BIOLOGY (2^{nd} ed., John Wiley and Sons, NY, 1994); and Hale and Marham, THE HARPER COLLINS DICTIONARY OF BIOLOGY (Harper Perennial, NY, 1991) provide those of skill in the art with a general dictionary of many of the terms used herein.

Also, as used herein, the singular terms "a", "an," and "the" include the plural reference unless the context clearly indicates otherwise. Unless otherwise indicated, nucleic acids are written left to right in 5' to 3' orientation; amino acid sequences are written left to right in amino to carboxy orientation, respectively. It is to be understood that the compositions, compounds and methods is not limited to the particular methodology, protocols, and reagents described, as these may vary, depending upon the context they are used by those of skill in the art.

It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The term "bleaching," as used herein, means the process of treating textile materials such as a fiber, yarn, fabric, garment and non-wovens to produce a lighter color in said fiber, yarn, fabric, garment or non-wovens. For example, bleaching as used herein means the whitening of the fabric by removal, modification or masking of color-causing compounds in cellulosic or other textile materials. Thus, "bleaching" refers to the treatment of a textile for a sufficient length of time and under appropriate pH and temperature conditions to effect a brightening (*i.e*., whitening) of the textile. Bleaching may be performed using chemical bleaching agent and/or enzymatically generated bleaching agents. Examples of suitable bleaching agents include, but are not limited to, ClO₂, H₂O₂, peracids, NO₂, etc. In the present processes, methods and compositions, peroxides such as H₂O₂ and peracids are preferably generated enzymatically.

The term "bleaching agent" as used herein encompasses any moiety that is capable of bleaching fabrics.

"Chemical bleaching agent(s)" are entities that are capable of bleaching a textile without the presence of an enzyme. They may require the presence of a bleach activator. Examples of suitable chemical bleaching agents useful in the processes, methods and compositions described herein are sodium peroxide, sodium perborate, potassium permanganate, other peracids. In some aspects, H₂O₂ may be considered a chemical bleaching agent when it has not been generated enzymatically *in situ*.

The term "one-step textile processing composition" refers to a preparation comprising at least one bioscouring enzyme and at least one enzymatically generated bleaching agent, desizing agent, and/or dyeing agent. Thus, the processing composition may further comprises at least one desizing enzyme. The one-step textile processing composition will contain sufficient enzymes to provide the enzyme levels provided for herein in the treatment liquor, i.e., the aqueous medium. Enzymes useful herein are wild-type enzymes as well as variants thereof. Preferably, the variants have been engineered to be oxidatively stable, e.g, stable in the presence of hydrogen peroxide and/or in the absence of divalent ions.

The phrase "enzymatic bleaching system" means enzymes and substrates capable of enzymatically generating a bleaching agent. An enzymatic bleaching system may comprise an ester source, an acyl transferase (or perhydrolase) and a hydrogen peroxide source.

"Ester source" refers to perhydrolase substrates that contain an ester linkage. Esters comprising aliphatic and/or aromatic carboxylic acids and alcohols are utilized with the perhydrolase enzymes. The ester source may be an acetate ester, or selected from one or more of propylene glycol diacetate, ethylene glycol diacetate, triacetin, ethyl acetate and tributyrin. The ester sources may be selected from the esters of one or more of the following acids: formic acid, acetic acid, propionic acid, butyric acid, valeric acid, caproic acid, caprylic acid, nonanoic acid, decanoic acid, dodecanoic acid, myristic acid, palmitic acid, stearic acid, and oleic acid.

The term "hydrogen peroxide source" means hydrogen peroxide that is added to the textile treatment bath either from an exogenous (*i.e*., an external or outside) source or generated in situ by the action of an hydrogen peroxide generating oxidase on a its substrate.

The term "hydrogen peroxide generating oxidase" means an enzyme that catalyzes an oxidation/reduction reaction involving molecular oxygen (O₂) as the electron acceptor. In these reactions, oxygen is reduced to water (H₂O) or hydrogen peroxide (H₂O₂). Oxidases suitable for use herein are the oxidases that generate hydrogen peroxide (as opposed to water) on its substrate. An example of a hydrogen peroxide generating oxidase and its substrate suitable for use herein would be glucose oxidase and glucose. Other enzymes (*e.g.*, alcohol oxidase, ethylene glycol oxidase, glycerol oxidase, amino acid oxidase, etc.) that can generate hydrogen peroxide also find use with ester substrates in combination with the perhydrolase enzymes to generate peracids. In some embodiments, the hydrogen peroxide generating oxidase is a carbohydrate oxidase.

As used herein, "textile" refers fibers, yarns, fabrics, garments, and non-wovens. The term encompasses textiles made from natural, synthetic (*e.g.*, manufactured), and various natural and synthetic blends. Thus, the term "textile(s)" refers to unprocessed and processed fibers, yarns, woven or knit fabrics, non-wovens, and garments. In the present specification, the terms "textile(s)," "fabric(s)," and "garment(s)" will be interchangeable unless expressly provided otherwise. The term "textile(s) in need of processing" refers to textiles that need to be desized and/or scoured and/or bleached or may be in need of other treatments, such as biopolishing.

The term "textile(s) in need of bleaching" refers to textiles that need to be bleached without reference to other possible treatments. These textiles may or may not have been already subjected to other treatments. Similarly, these textiles may or may not need subsequent treatments.

As used herein, "textile materials" is a general term for fibers, yarn intermediates, yarns, fabrics, products made from fabrics (*e.g.*, garments and other articles) and non-wovens.

As used herein, the term "compatible," means that the components of a one-step textile processing composition do not reduce the enzymatic activity of the pectate lyase or other enzymes that may be present in the composition to such an extent that the pectate lyase is not effective as desired during normal use situations. Specific composition materials are exemplified in detail hereinafter.

As used herein, "effective amount of pectate lyase enzyme" refers to the quantity of pectate lyase enzyme necessary to achieve the enzymatic activity required in the processes or methods described herein. Such effective amounts are readily ascertained by one of ordinary skill in the art and are based on many factors, such as the particular enzyme variant used, the pH used, the temperature used and the like, as well as the results desired (*e.g.*, level of whiteness, dyeability, ability to desize).

As used herein, "oxidizing chemical" refers to a chemical that has the capability of bleaching a textile. The oxidizing chemical is present at an amount, pH and temperature suitable for bleaching. The term includes, but is not limited to hydrogen peroxide, chlorine bleaches, and peracids.

As used herein, the term "enzymatic conversion" refers to the modification of a substrate to an intermediate or the modification of an intermediate to an end-product by contacting the substrate or intermediate with an enzyme. Contact may be made by directly exposing the substrate or intermediate to the appropriate enzyme.

As used herein, the phrase, "stability to proteolysis" refers to the ability of a protein (*e.g.*, an enzyme) to withstand proteolysis. It is not intended that the term be limited to the use of any particular protease to assess the stability of a protein.

As used herein, "oxidative stability" refers to the ability of a protein to function under oxidative conditions. In particular, the term refers to the ability of a protein to function in the presence of various concentrations of H₂O₂ and/or peracid. Stability under various oxidative conditions can be measured either by standard procedures known to those in the art and/or by the methods described herein. A substantial change in oxidative stability is evidenced by at least about a 5% or greater increase or decrease (in most embodiments, it is preferably an increase) in the half-life of the enzymatic activity, as compared to the enzymatic activity present in the absence of oxidative compounds.

As used herein, "pH stability" refers to the ability of a protein to function at a particular pH. In general, most enzymes have a finite pH range at which they will function. In addition to enzymes that function in mid-range pHs *(i.e.,* around pH 7), there are enzymes that are capable of working under conditions with very high or very low pHs. Stability at various pHs can be measured either by standard procedures known to those in the art and/or by the methods described herein. A substantial change in pH stability is evidenced by at least about 5% or greater increase or decrease (in most embodiments, it is preferably an increase) in the half-life of the enzymatic activity, as compared to the enzymatic activity at the enzyme's optimum pH. However, it is not intended that the present processes, methods and/or compositions described herein be limited to any pH stability level nor pH range.

As used herein, "thermal stability" refers to the ability of a protein to function at a particular temperature. In general, most enzymes have a finite range of temperatures at which they will function. In addition to enzymes that work in mid-range temperatures (*e.g.*, room temperature), there are enzymes that are capable of working in very high or very low temperatures. Thermal stability can be measured either by known procedures or by the methods described herein. A substantial change in thermal stability is evidenced by at least about 5% or greater increase or decrease (in most embodiments, it is preferably an increase) in the half-life of the catalytic activity of a mutant when exposed to a different temperature (*i.e*., higher or lower) than optimum temperature for enzymatic activity. However, it is not intended that the processes, methods and/or compositions described herein be limited to any temperature stability level nor temperature range.

As used herein, the term "chemical stability" refers to the stability of a protein (*e.g.*, an enzyme) towards chemicals that adversely affect its activity. Such chemicals include, but are not limited to hydrogen peroxide, peracids, anionic surfactants, cationic surfactants, non-ionic surfactants, chelants, etc. However, it is not intended that the processes, methods and/or compositions described herein be limited to any particular chemical stability level nor range of chemical stability.

As used herein, the terms "purified" and "isolated" refer to the removal of contaminants from a sample. For example, pectate lyases are purified by removal of contaminating proteins and other compounds within a solution or preparation that are not pectate lyases. Recombinant pectate lyases may be expressed in bacterial or fungal host cells and these recombinant pectate lyases are purified by the removal of other host cell constituents; the percent of recombinant pectate lyases polypeptides is thereby increased in the sample.

A "purified preparation" or a "substantially pure preparation" of a polypeptide (such as an enzyme), as used herein, means a polypeptide that has been separated from other proteins, lipids, and nucleic acids with which it naturally occurs. Preferably, the polypeptide is also separated from substances, *e.g.,* antibodies or gel matrix (*e.g.,* polyacrylamide), which are used to purify it. Preferably, the polypeptide constitutes at least 10, 20, 50 70, 80 or 95% dry weight of the purified preparation. The enzymes may be used or supplied as a purified preparation.

As used herein, "protein" refers to any composition comprised of amino acids and recognized as a protein by those of skill in the art. The terms "protein," "peptide" and "polypeptide" are used interchangeably herein. Wherein a peptide is a portion of a protein, those skilled in the art understand the use of the term in context.

As used herein, functionally and/or structurally similar proteins are considered to be "related proteins." These proteins may be derived from a different genus and/or species, including differences between classes of organisms (*e.g*., a bacterial protein and a fungal protein). These proteins may be derived from a different genus and/or species, including differences between classes of organisms (*e.g.*, a bacterial enzyme and a fungal enzyme). Related proteins may be provided from the same species. Indeed, it is not intended that the processes, methods and/or compositions described herein be limited to related proteins from any particular source(s). In addition, the term "related proteins" encompasses tertiary structural homologs and primary sequence homologs. The term may be encompasse proteins that are immunologically cross-reactive.

As used herein, the term "derivative" refers to a protein that is derived from a protein by addition of one or more amino acids to either or both the C- and N-terminal end(s), substitution of one or more amino acids at one or a number of different sites in the amino acid sequence, and/or deletion of one or more amino acids at either or both ends of the protein or at one or more sites in the amino acid sequence, and/or insertion of one or more amino acids at one or more sites in the amino acid sequence. The preparation of a protein derivative can be achieved by modifying a DNA sequence, which encodes for the native protein, transformation of that DNA sequence into a suitable host, and expression of the modified DNA sequence to form the derivative protein.

Related (and derivative) proteins comprise "variant proteins." Variant proteins may differ from a parent protein, e.g., a wild-type protein, and one another by a small number of amino acid residues. The number of differing amino acid residues may be one or more, preferably 1, 2, 3, 4, 5, 10, 15, 20, 30, 40, 50, or more amino acid residues. The number of different amino acids between variants is between 1 and 10. In some aspects, related proteins and particularly variant proteins comprise at least 35%, 40%, 45%, 50%, 55%, 60%, 65%, 70%, 75%, 80%, 85%, 90%, 95%, 97%, 98%, or 99% amino acid sequence identity. Additionally, a related protein or a variant protein as used herein, refers to a protein that differs from another related protein or a parent protein in the number of prominent regions. For example, in some embodiments, variant proteins have 1, 2, 3, 4, 5, or 10 corresponding prominent regions that differ from the parent protein.

Several methods are known in the art that are suitable for generating variants of the enzymes described herein, including but not limited to site-saturation mutagenesis, scanning mutagenesis, insertional mutagenesis, random mutagenesis, site-directed mutagenesis, and directed-evolution, as well as various other recombinatorial approaches.

Homologous proteins may be engineered to produce enzymes with the desired activity(ies).

As used herein, the term "analogous sequence" refers to a sequence within a protein that provides similar function, tertiary structure, and/or conserved residues as the protein of interest (*i.e.*, typically the original protein of interest). For example, in epitope regions that contain an alpha helix or a beta sheet structure, the replacement amino acids in the analogous sequence preferably maintain the same specific structure. The term also refers to nucleotide sequences, as well as amino acid sequences. Analogous sequences may be developed such that the replacement amino acids result in a variant enzyme showing a similar or improved function. The tertiary structure and/or conserved residues of the amino acids in the protein of interest may be located at or near the segment or fragment of interest. Thus, where the segment or fragment of interest contains, for example, an alpha-helix or a betasheet structure, the replacement amino acids preferably maintain that specific structure.

As used herein, "homologous protein" refers to a protein (*e.g.*, pectate lyase) that has similar action and/or structure, as a protein of interest (*e.g.*, a pectate lyase from another source). It is not intended that homologs be necessarily related evolutionarily. Thus, it is intended that the term encompass the same or similar enzyme(s) (*i.e.,* in terms of structure and function) obtained from different species. It may be desirable to identify a homolog that has a quaternary, tertiary and/or primary structure similar to the protein of interest, as replacement for the segment or fragment in the protein of interest with an analogous segment from the homolog will reduce the disruptiveness of the change. Homologous proteins may induce similar immunological response(s) as a protein of interest.

As used herein, "wild-type" and "native" proteins are those found in nature. The terms "wild-type sequence," and "wild-type gene" are used interchangeably herein, to refer to a sequence that is native or naturally occurring in a host cell. The wild-type sequence may refer to a sequence of interest that is the starting point of a protein engineering project. The genes encoding the naturally-occurring protein may be obtained in accord with the general methods known to those skilled in the art. The methods generally comprise synthesizing labeled probes having putative sequences encoding regions of the protein of interest, preparing genomic libraries from organisms expressing the protein, and screening the libraries for the gene of interest by hybridization to the probes. Positively hybridizing clones are then mapped and sequenced.

The degree of homology between sequences may be determined using any suitable method known in the art (*See e.g*., Smith and Waterman, 2 ADV. APPL. MATH.482 (1981); Needleman and Wunsch, 48 J. MOL. BIOL. 443 (1970); Pearson and Lipman, 85 PROC. NAT'L. ACAD. SCI. USA 2444 (1988); programs such as GAP, BESTFIT, FASTA, and TFASTA in the Wisconsin Genetics Software Package (Genetics Computer Group, Madison, WI); and Devereux *et al.,* 12 NUCL. ACID RES. 387-395 (1984)).

For example, PILEUP is a useful program to determine sequence homology levels. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle, (Feng and Doolittle, 35 J. MOL. EVOL. 351-360 (1987)). The method is similar to that described by Higgins and Sharp (Higgins and Sharp, 5 CABIOS 151-153 (1989)). Useful PILEUP parameters including a default gap weight of 3.00, a default gap length weight of 0.10, and weighted end gaps. Another example of a useful algorithm is the BLAST algorithm, described by Altschul *et al.,* (Altschul et al., 215 J. MOL. BIOL. 403-410 (1990); and Karlin et al., PROC. NAT'L. ACAD. SCI. USA 90:5873-5787 (1993)). One particularly useful BLAST program is the WU-BLAST-2 program (*See*, Altschul et al., 266 METH. ENZYMOL. 460-480 (1996)). parameters "W," "T," and "X" determine the sensitivity and speed of the alignment. The BLAST program uses as defaults a word length (W) of 11, the BLOSUM62 scoring matrix (*See,* Henikoff and Henikoff, 89 PROC. NAT'L. ACAD. SCI. USA 10915 (1989)) alignments (B) of 50, expectation (E) of 10, M'5, N'-4, and a comparison of both strands.

The phrases "substantially similar and "substantially identical" in the context of at least two nucleic acids or polypeptides typically means that a polynucleotide or polypeptide comprises a sequence that has at least about 40% identity, more preferable at least about 50% identity, yet more preferably at least about 60% identity, preferably at least about 75% identity, more preferably at least about 80% identity, yet more preferably at least about 90%, still more preferably about 95%, most preferably about 97% identity, sometimes as much as about 98% and about 99% sequence identity, compared to the reference *(i.e.,* wild-type) sequence. Sequence identity may be determined using known programs such as BLAST, ALIGN, and CLUSTAL using standard parameters. (*See e.g*., Altschul, et al., 215 J. MOL. BIOL. 403-410 (1990); Henikoff et al., 89 PROC. NAT'L. ACAD. SCI. USA 10915 (1989); Karin et al., 90 PROC. NAT'L. ACAD. SCI. USA 90: 5873 (1993); and Higgins et al., 73 GENE 73: 237-244 (1988)). Software for performing BLAST analyses is publicly available through the National Center for Biotechnology Information. Databases may be searched using FASTA (Pearson et al., 85 PROC. NAT'L. ACAD. SCI. USA 2444-2448 (1988)). One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution. Another indication that two nucleic acid sequences are substantially identical is that the two molecules hybridize to each other under stringent conditions (*e.g.*, within a range of medium to high stringency).

The term "simultaneously" or "simultaneous" or "one-step" are intended to indicate that at least a portion (*e.g.*, preferably about 75 % or more, more preferably about 90 % or more) of the desizing, scouring, bleaching, and dyeing (and combinations of those steps) are carried out in a single operation. The term is not intended to mean that the textiles treated by the methods and compositions cannot be treated more than once. Rather, the term means that for each treatment cycle, multiple components, as detailed elsewhere in this application, are used in processing the textile at one time. Likewise, the components of the treatment may be added one at a time, all at once or in groups providing that for at least a portion of the treatment cycle all of the components are present. The portion of the treatment cycle in which all of the components are present may vary depending on the total length of the treatment cycle.

The term "simultaneously" is also intended to indicate in some embodiments that at least a portion of the bioscouring and enzymatic bleaching are carried out in a single operation. This has the obvious advantage that the washing and other treatments normally performed between separately conducted scouring, bleaching, and/or dyeing steps are no longer required. Thereby, the water, time and energy demand as well as the demand to different equipment to be used for each of the processes are considerably reduced. Furthermore, depending on the type of fabric to be treated and the nature of impurities present thereon, a desizing effect may be obtained during the performance of the processes discussed herein. Thus, in such cases, no additional desizing treatment needs to be performed. While it is preferred that all de-sizing be carried out in conjunction with the bleaching step, one of ordinary skill in the art will recognize that some portion of de-sizing may be carried out separately from the bleaching step. The terms "size" or "sizing" refer to compounds used in the textile industry to improve weaving performance by increasing the abrasion resistance and strength of the yarn. Size is usually made of, for example, starch or starch-like compounds.

The terms "desize" or "desizing," as used herein, refer to the process of eliminating size, generally starch, from textiles usually prior to applying special finishes, dyes or bleaches.

"Desizing enzyme(s)" as used herein refer to enzymes that are used to enzymatically remove the size. Exemplary enzymes are amylases and mannanases.

The terms "bioscouring" as used herein, means to remove impurities, for example, much of the non-cellulosic compounds (*e.g*., pectins, proteins, wax, and motes, etc) naturally found in cotton or other textiles. In addition to the natural non-cellulosic impurities, scouring can remove, in some embodiments, residual manufacturing introduced materials such as spinning, coning or slashing lubricants. Scouring encompasses bioscouring, but also includes the use of harsh chemical scouring agents such as sodium hydroxide.

The term "bioscouring enzyme(s)" therefore refers to an enzyme(s) capable of removing at least a portion of the impurities found in cotton or other textiles.

The term "motes" refers to unwanted impurities, such as cotton seed fragments, leaves, stems and other plant parts, which cling to the fiber even after mechanical ginning process.

The term "greige" (pronounced gray) textiles, as used herein, refer to textiles that have not received any bleaching, dyeing or finishing treatment after being produced. For example, any woven or knit fabric off the loom that has not yet been finished (desized, scoured, etc.), bleached or dyed is termed a greige textile. The textiles used in the examples, *infra*, are greige textiles.

The term "dyeing," as used herein, refers to applying a color, especially by soaking in a coloring solution, to, for example, textiles.

The term "non-cotton cellulosic" fiber, yarn or fabric means fibers, yarns or fabrics which are comprised primarily of a cellulose based composition other than cotton. Examples of such compositions include linen, ramie, jute, flax, rayon, lyocell, cellulose acetate and other similar compositions which are derived from non-cotton cellulosics.

The term "protease" means a protein or polypeptide domain of a protein or polypeptide derived from a microorganism, *e.g.* a fungus, bacterium, or from a plant or animal, and that has the ability to catalyze cleavage of peptide bonds at one or more of various positions of a protein carbohydrate backbone.

The term "cutinase," as used herein, refers to as a plant, bacterial or fungal derived enzyme used in textile processing. Cutinases are lipolytic enzymes capable of hydrolyzing the substrate cutin. Cutinases can breakdown fatty acid esters and other oil-based compositions need to be removed in the processing (*e.g.*, the scouring) of textiles. "Cutinase" means an enzyme that has significant plant cutin hydrolysis activity. Specifically, a cutinase will have hydrolytic activity on the biopolyester polymer cutin found on the leaves of plants. Suitable cutinases may be isolated from many different plant, fungal and bacterial sources. Examples of cutinases are provided in LIPASES: STRUCTURE, MECHANISM AND GENETIC ENGINEERING, 71-77 (VCH Publishers, edited by Alberghina, Schmid & Verger, 1991); LIPASES, 471-77 (Elsevier, edited by Borgstrom & Brockman, 1984); and Sebastian et al., 169 J. BACTERIOLOGY 131-136 (1987).

The term "pectate lyase," as used herein, refers to a type of pectinase that cleaves pectate to give oligosaccharides with 4-deoxy-alpha-D-gluc-4-enuronosyl groups at their non-reducing ends. "Pectinases" are a group of enzymes that cleave glycosidic linkages of pectic substances, mainly poly(1,4-alpha-D-galacturonide and its derivatives (*see* Sakai et al., "Pectin, pectinase and protopectinase: production, properties and applications," in 39 ADVANCES IN APPLIED MICROBIOLOGY 213-294 (1993)). Pectate lyase is also referred to as polygalacturonate lyase (EC 4.2.2.2) (PGL) and poly(1,4-alpha-D-galacturonide) lyase. By "*B. subtilis* pectate lyase" is meant to include the polypeptide of SEQ ID NO: 1, as well as variants thereof that are characterized as having a pH range of about 6 to about 8 when using raw cotton as a substrate, a pI of about 7.3, a temperature range of about 15°C to about 90°C and is about 43 kD. It should be noted that the pH range can vary depending on the substrate used. Other temperature ranges that can be used include from about 20°C to about 75°C, and more preferably from about 25°C to about 60°C. Thus, variants can include pectate lyases obtained from other *B. subtilis* strains.

The term "pectin" denotes pectate, polygalacturonic acid and pectin that may be esterified to a higher or lower degree.

The term "α-amylase," as used herein, refers to an enzyme that cleaves the α (1-4)glycosidic linkages of amylose to yield maltose molecules (disaccharides of α-glucose). Amylases are digestive enzymes found in saliva and are also produced by many plants. Amylases break down long-chain carbohydrates (such as starch) into smaller units. An "oxidative stable" α-amylase is an α-amylase that is resistive to degradation by oxidative means, when compared to non-oxidative stable α-amylase, especially when compared to the non-oxidative stable α-amylase form which the oxidative stable α-amylase was derived.

As used herein, "microorganism" refers to a bacterium, a fungus, a virus, a protozoan, and other microbes or microscopic organisms.

As used herein, a substance (*e.g.*, a polynucleotide or protein) "derived from" a microorganism means that the substance is native to the microorganism.

### 1.2 Abbreviations

The following abbreviations are used herein and refer to the associate words and phrases:
- AATCC: American Association of Textile Chemists and Colorists
- APSU: alkaline pectinase standard unit
- ATCC: American Type Culture Collection
- bp: base pairs
- CBG: cellobiohydrolase
- CMC: carboxymethylcellulose
- CWDE: cell wall degrading enzyme
- EC: Enzyme Class
- ECU: endo-cellulose units
- EDTA: ethylenediaminetetraacetate
- EGTA: ethylene glycol-*O*,-*O'*-bis(2-amino-ethyl)-*N*,*N*,*N'*,*N'*-tetraacetic acid
- FPLC: fast protein liquid chromatography
- HPLC: high performance liquid chromatography
- kDa: kilo Dalton
- Kₘ: Michaelis-Menten constant
- pI: isoelectric point
- ppm: parts per million
- PVA: polyvinyl alcohol
- RAU: reference amylase unit
- SDS-PAGE: sodium dodecyl sulfate polyacrylamide gel electrophoresis
- sp.: Species as in microorganism
- TAU: thermostable amylase unit
- TSAU: textile stearothermophilus amylase unit
- TTAU: textile thermostable amylase unit.
- Vₘₐₓ: symbol for maximum velocity (*i.e.* limiting rate) of an enzyme reaction
- w/v: percentage weight of a substance of the total volume
- w/w: by weight

### 2. Isolation and Characterization of B. subtilis Pectate Lyase

A pectate lyases (*pel*) was obtained from *Bacillus subtilis* subsp. *subtilis* str. 168. The protein was then over-expressed in *B. subtilis.* The expressed protein has an amino acid sequence of SEQ ID NO: 1. The *B. subtilis* pectate lyase is encoded by the *pel* gene (SEQ ID NO: 2):

5'-GCTGATTTAGGCCACCAGACGTTGGGATCCAATGATGGCTGGGGCGCGTACTCGA
CCGGCACGACAGGCGGATCAAAAGCATCCTCCTCAAATGTGTATACCGTCAGCAAC
AGAAACCAGCTTGTCTCGGCATTAGGGAAGGAAACGAACACAACGCCAAAAATCAT
TTATATCAAGGGAACGATTGACATGAACGTGGATGACAATCTGAAGCCGCTTGGCC
TAAATGACTATAAAGATCCGGAGTATGATTTGGACAAATATTTGAAAGCCTATGATC
CTAGCACATGGGGCAAAAAAGAGCCGTCGGAACACAAGAAGCGAGAGCACG
CTCTCAGAAAAACCAAAAAGCACGGGTCATGGTGGATATCCCTGCAAACACGACGA
TCGTCGGTTCAGGGACTAACGCTAAAGTCGTGGGAGGAAACTTCCAAATCAAGAGT
GATAACGTCATTATTCGCAACATTGAATTCCAGGATGCCTATGACTATTTTCCGCAA
TGGGATCCGACTGACGGAAGCTCAGGGAACTGGAACTCACAATACGACAACATCAC
GATAAACGGCGGCACACACATCTGGATTGATCACTGTACATTTAATGACGGTTCGC

GTCCGGACAGCACATCACCGAAATATTATGGAAGAAAATATCAGCACCATGACGGC
CAAACGGATGCTTCCAACGGTGCTAACTATATCACGATGTCCTACAACTATTATCAC
GATCATGATAAAAGCTCCATTTTCGGATCAAGTGACAGCAAAACCTCCGATGACGG
CAAATTAAAAATTACGCTGCATCATAACCGCTATAAAAATATTGTCCAGCGCGCGC
CGAGAGTCCGCTTCGGGCAAGTGCACGTATACAACAACTATTATGAAGGAAGCACA
AGCTCTTCAAGTTATCCTTTTAGCTATGCATGGGGAATCGGAAAGTCATCTAAAATC
TATGCCCAAAACAATGTCATTGACGTACCGGACTGTCAGCTGCTAAAACGATCAG
CGTATTCAGCGGGGGAACGGCTTTATATGACTCCGGCACGTTGCTGAACGGCACAC
AGATCAACGCATCGGCTGCAAACGGGCTGAGCTCTTCTGTCGGCTGGACGCCGTCTC
TGCATGGATCGATTGATGCTTCTGCTAATGTGAAATCAAATGTTATAAATCAAGCGG
GTGCGGGTAAATTAAAT-3'.

The 420 amino acid pectate lyase will likely be classified in Enzyme Class EC 4.2.2.2. It has the following properties:

| | |
|---|---|
| Amino acids, DNA | 420 residues; 1260 base pairs |
| Molecular Weight | 45,497 |
| Function | pectate lyases (EC 4.2.2.2) |
| Protein cluster | IG hypothetical 16615 |

The mature protein has a calculated molecular weight of 43.4 kDa and pI of 7.3 based on reducing SDS-PAGE gel analysis. The mature protein has the following sequence (depicted in an amino to carboxy orientation): 
ADLGHQTLGSNDGWGAYSTGTTGGSKASSSNVYTVSNRNQLVSALGKETNTTPKIIYIK
GTIDMNVDDNIKPLGLNDYKDPEYDLDKYLKAYDPSTWGKKEPSGTQEEARARSQKN
QKARVMVDIPANTTIVGSGTNAKVVGGNFQIKSDVIIRNIEFQDAYDYFPQWDPTDGSS
GNWNSQUDNITINGGTHIWIDHCTFNDGSRPDSTSPKYYGRKYQHHDGQTDASNGANY
ITMSYNYYHDHDKSSIFGSSDSKTSDDGKLKITLHHNRYKNIVQRAPRVRFGQVHVYNN
YYEGSTSSSSYPFSYAWGIGKSSKIYAQNNVIDVPGLSAAKTISVFSGGTALYDSGTLLN
GTQINASAANGLSSSVGWTPSLHGSIDASANVKSNVINQAGAGKLN (SEQ ID NO: 1).
 The protein was expressed as a fusion protein fused to the AprE signal sequence. The signal sequence was then cleaved during secretion of the protein. Other signal sequences can be substituted.

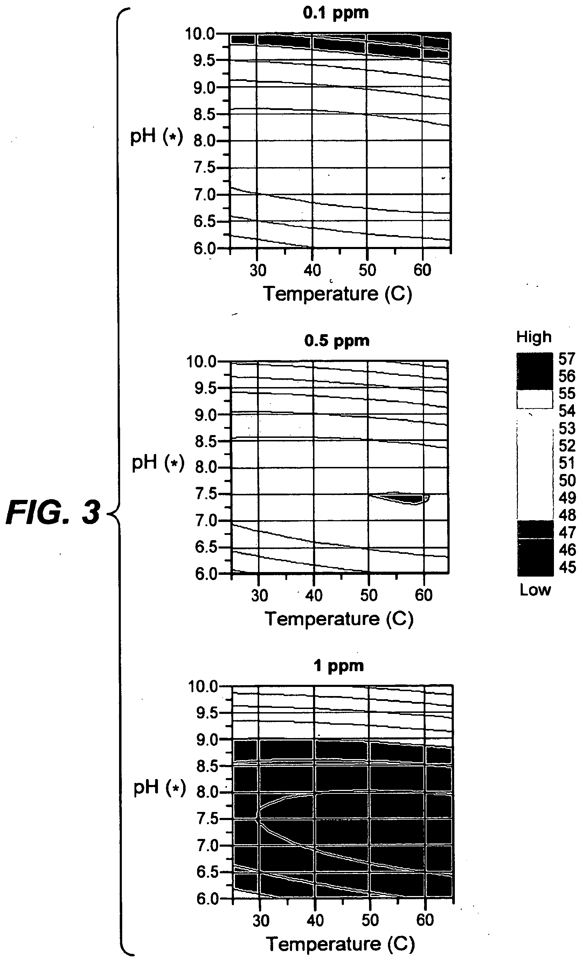

The *B. subtilis pel* gene was cloned into the *B. subtilis* strain BG3594comK as the host cell. Other cells can be substituted for expression of the gene. The mature protein produced by *B. subtilis* strain BG3594comK had a calculated molecular weight of 43.4 kDa and pI of 7.3. This protein has a broad pH range and a broad temperature range. FIG. 3. The optimal pH of the disclosed pectate lyase is lower than that of Bioprep™ 3000L (Novozymes, pH range of 7.5-8.5). *See* FIG. 1. Additionally, the optimal pH activity of the pectate lyase disclosed herein has activity over a broad temperature range *(i.e.,* 20°C to 65°C), unlike that of Bioprep 3000L which has a narrow temperature range in which it acts.

The pectate lyase disclosed herein has a dose response of pectin removal in large excess to that obtained by Bioprep 3000L. *See* Fig. 2. The disclosed pectate lyase had a lower thermal stability at 55°C as compared to that of Bioprep 3000L. However, the enzyme is optimally used at room temperature. The disclosed pectate lyase has EDTA stability that is improved over Bioprep 3000L when tested at room temperature.

The pectate lyase includes homologs and can be expressed in various suitable microorganisms and host cells. Preferably the host cell expressing SEQ ID NO: 1 or variants thereof, have increased expression suitable for large scale expression of the polypeptide.

Pectate lyase activity of variants can be measured by applying a test solution to 4 mm holes punched out in agar plates (such as, for example, LB agar), containing 0.7 % w/v sodium polygalacturonate (Sigma P 1879). The plates are then incubated for 6 h at a particular temperature (such as, *e.g.,* 75°C). The plates are then soaked in either (i) 1 M CaCl₂ for 0.5 h or (ii) 1% mixed alkyl trimethylammonium Br (MTAB, Sigma M-7635) for 1 h. Both of these procedures cause the precipitation of polygalacturonate within the agar. Pectate lyase activity can be detected by the appearance of clear zones within a background of precipitated polygalacturonate. Sensitivity of the assay is calibrated using dilutions of a standard preparation of pectate lyase.

### 3. Compositions Comprising Pectate Lyase

Described herein is an enzyme composition or preparation comprising a purified pectate lyase of *B. subtilis* or variants thereof. The pectate lyase can be used alone, or in combination with other polypeptides having enzymatic activity. Preferable compositions include bioscouring, bleaching, desizing, and/or dyeing compositions. More preferred are one-step compositions that achieve bioscouring, bleaching, desizing, and/or dyeing and combinations thereof in one step of a textile. Additional compositions include cleaning compositions, such as detergents for the cleaning of textiles, which comprise pectate lyase.

The disclosed pectate lyases can also be utilized in enzymatic scouring (bioscouring) processes in the preparation of cellulosic material, *e.g.,* for proper response in subsequent bleaching and/or dyeing operations. The enzymes can also be utilized for removing certain soils or stains present on laundry, especially soils and spots resulting from, for example, galactan or arabinogalactan containing food, plants and the like. The pectate lyase enzymes can also be used in cleaning solutions and reagents to clean hard surfaces by having the effect of removing or assisting in the removal of certain soils or stains from hard surfaces.

Thus, compositions can include *inter alia* desizing enzymes, bioscouring enzymes (in addition to pectate lyase), bleaching agents, and dyeing agents.

### 3.1 Desizing Enzymes

The compositions described herein can include desizing enzymes in combination with a pectate lyase. Desizing agents can be used with bleaching agents, bioscouring enzymes and biopolishing enzymes in addition to pectate lyase, and/or dyeing agents. Any suitable desizing enzyme may be used. Preferably, the desizing enzyme is an amylolytic enzyme. Mannanases and glucoamylases also find use herein. More preferably, the desizing enzyme is a α- or β-amylase and combinations thereof.

### 3.1.1 Amylases

Alpha and beta amylases can be used and include those of bacterial or fungal origin. Chemically or genetically modified mutants of such amylases are also contemplated. Preferred α-amylases include, for example, α-amylases obtainable from *Bacillus* species. Useful amylases include but are not limited to: Optisize 40, Optisize 160, Optisize HT 260, Optisize HT 520, Optisize HT Plus, Optisize FLEX (all from Genencor Int. Inc.), Duramyl^{™}, Termamyl^{™}, Fungamyl^{™}, and BAN^{™} (all available from Novozymes A/S, Bagsvaerd, Denmark). Other preferred amylolytic enzymes are CGTases (cyclodextrin glucanotransferases, EC 2.4.1.19), *e*.*g*., those obtained from species of *Bacillus*, *Thermoanaerobactor* or *Thermoanaerobacterium*.

The activity of Optisize 40 and Optisize 160 is expressed in reference amylase unit (RAU) per gram (RAU/g) of product. One RAU is the amount of enzyme that will convert 1 gram of starch into soluble sugars in one hour under standard conditions. The activity of Optisize HT 260, Optisize HT 520, and Optisize HT Plus is expressed in textile thermostable amylase unit per gram (TTAU/g). One TTAU is the amount of enzyme that is needed to hydrolyze 100 mg of starch into soluble sugars per hour under standard conditions. The activity of Optisize FLEX is determined in textile stearothermophilus amylase units per gram (TSAU/g). One TSAU is the amount of enzyme needed to convert 1 mg of starch into soluble sugars in one minute under standard conditions. Dosage of the amylase varies depending on the process type. Smaller dosages would require more time than larger dosages of the same enzyme. However, there is no upper limit on the amount of desizing amylase other than what may be dictated by the physical characteristics of the solution. Excess enzyme does not hurt the fabric; it allows for a shorter processing time. Based on the foregoing and the enzyme utilized the following minimum dosages for desizing are suggested:

| Amylase Product | Minimum dosage (per liter of desizing liquor) | Typical Range (per liter of desizing liquor) |
|---|---|---|
| Optisize 40 | 1,000 RAU | 2,000-70,000 RAU |
| Optisize 160 | 1,000 RAU | 2,000-70,000 RAU |
| Optisize HT 260 | 1,000 TTAU | 3,000-100,000 TTAU |
| Optisize HT 520 | 1,000 TTAU | 3,000-100,000 TTAU |
| Optisize HT Plus | 1,000 TTAU | 3,000-100,000 TTAU |
| Optisize FLEX | 5,000 TSAU | 13,000-65,000 TSAU |

The desizing enzymes may also preferably be derived from the enzymes listed above in which one or more amino acids have been added, deleted, or substituted, including hybrid polypeptides, so long as the resulting polypeptides exhibit desizing activity. Such variants can be created using conventional mutagenesis procedures and identified using, *e*.*g*., high-throughput screening techniques such as the agar plate screening procedure.

The desizing enzyme is added to the aqueous solution *(i.e.,* the treating composition) in an amount effective to desize the textile materials. Typically, desizing enzymes, such as α-amylases, are incorporated into the treating composition in amount from 0.00001% to 2% of enzyme protein by weight of the fabric, preferably in an amount from 0.0001% to 1% of enzyme protein by weight of the fabric, more preferably in an amount from 0.001% to 0.5% of enzyme protein by weight of the fabric, and even more preferably in an amount from 0.01% to about 0.2% of enzyme protein by weight of the fabric.

### 3.2 Bioscouring Enzymes

One aspect contemplates the use of pectate lyase as the only bioscouring agent. Another aspect contemplates its use in combination with one or more additional bioscouring enzymes. The bioscouring enzymes can comprise a suitable bioscouring composition, or a one-step composition that does one or more of bioscouring, bleaching, desizing, and/or dyeing. Other bioscouring enzymes that can be used in conjunction with the disclosed pectate lyase and variants thereof, include but are not limited to other pectinases including other pectate lyases, and cutinases, proteases, lipases and cellulases. The bioscouring enzymes can be used with buffers including but not limited to phosphate, silicate, carbonate or citrate buffers, along with other auxiliary chemicals, such as but not limited to wetting agents, surfactants, emulsifiers, and chelating agents to enhance wetting properties of the textile.

Depending on the process chosen to be treated, different temperatures can be used. For cold pad batch processes, the scouring treatment can be done for example at about 15°C to about 45°C, and preferably from about 25°C to 35°C; for continuous, semi-continuous, and other batch processes, the process can be done at about 15°C to about 90°C, preferably from about 20°C and 75°C, and more preferably from about 25°C to about 60°C. To enhance scouring performances, auxiliary agents including but not limited to wetting agents, surfactants, emulsifiers, chelating agents could be used alone or in combination with other agents.

### 3.2.1 Pectinases

Any pectinolytic enzyme composition with the ability to degrade the pectin composition of, *e.g.,* plant cell walls, may be used. Suitable pectinases include, without limitation, those of fungal or bacterial origin. Chemically or genetically modified pectinases are also encompassed. Preferably, the pectinases are recombinantly produced or of natural origin. They may be mono-component enzymes.

Pectinases can be classified according to their preferential substrate, highly methyl-esterified pectin or low methyl-esterified pectin and polygalacturonic acid (pectate), and their reaction mechanism, β-elimination or hydrolysis. Pectinases can be mainly endo-acting, cutting the polymer at random sites within the chain to give a mixture of oligomers, or they may be exo-acting, attacking from one end of the polymer and producing monomers or dimers. Several pectinase activities acting on the smooth regions of pectin are included in the classification of enzymes provided by Enzyme Nomenclature (1992), *e.g.,* pectate lyase (EC 4.2.2.2), pectin lyase (EC 4.2.2.10), polygalacturonase (EC 3.2.1.15), exo-polygalacturonase (EC 3.2.1.67), exo-polygalacturonate-lyase (EC 4.2.2.9) and exo-poly-alpha-galacturonosidase (EC 3.2.1.82). In preferred embodiments, the methods utilize pectate lyases.

Pectate lyase enzymatic activity as used herein refers to catalysis of the random cleavage of α-1,4-glycosidic linkages in pectic acid (also called polygalcturonic acid) by transelimination. Pectate lyases are also termed polygalacturonate lyases and poly(1,4-D-galacturonide) lyases.

For purposes herein, pectate lyase enzymatic activity is the activity determined by measuring the increase in absorbance at 235 nm of a 0.1 % w/v solution of sodium polygalacturonate in 0.1 M glycine buffer at pH 10 (*see* Collmer et al., "Assay methods for pectic enzymes," in 161 METHODS ENZYMOL. 329-335 (1988)). Enzyme activity is typically expressed as x mol/min, *i.e.,* the amount of enzyme that catalyzes the formation of x mole product/min. An alternative assay measures the decrease in viscosity of a 5 % w/v solution of sodium polygalacturonate in 0.1 M glycine buffer at pH 10, as measured by vibration viscometry (APSU units). APSU unit assay is a viscosity measurement using the substrate polygalacturonic acid and can be performed as discussed in for example WO 2006/002034.

Additional pectate lyases that can bemused with the claimed *B*. *subtilis* pectate lyase and variants thereof include pectate lyases that have been cloned from different bacterial genera such as *Erwinia*, *Pseudomonas*, other *Bacillus* species and strains, *Klebsiella*, and *Xanthomonas*. Additional pectate lyases suitable for use herein are from *Bacillus subtilis* (Nasser, et al., 335 FEBS LETTS. 319-326 (1993)) and *Bacillus* sp. YA-14 (Kim, et al., 58 BIOSCI. BIOTECH. BIOCHEM. 947-949 (1994)). Other pectate lyases contemplated include those produced by *Bacillus pumilus* (Dave and Vaughn, 108 J. BACTERIOL. 166-174 (1971)), *B. polymyxa* (Nagel and Vaughn, 93 ARCH. BIOCHEM. BIOPHYS. 344-352 (1961)), *B. stearothermophilus* (Karbassi and Vaughn, 26 CAN. J. MICROBIOL. 26: 377-384 (1980)), *Bacillus* sp. (Hasegawa and Nagel, 31 J. FOOD SCI. 838-845 (1966)), and *Bacillus* sp. RK9 (Kelly and Fogarty, 24 CAN. J. MICROBIOL. 1164-1172 (1978)) have also been described and are contemplated to be used in the present compositions and methods in combination with *B. subtilis* pectate lyase and its variants as disclosed herein. Additional pectate lyases contemplated for use in combination include those disclosed in WO 04/090099 (Diversa) and WO 03/095638 (Novozymes).

An effective amount of pectolytic enzyme to be used depends on many factors, but can include a concentration of a pectolytic enzyme in the aqueous medium from about 0.0001% to about 1% microgram enzyme protein by weight of the fabric, preferably 0.0005% to 0.2% enzyme protein by weight of the fabric, more preferably 0.001% to about 0.05% enzyme protein by weight of the fabric.

### 3.2.2 Cutinases

Any cutinase suitable that may be used in the compositions and methods herein include, for example, the cutinase derived from *Humicola insolens* cutinase strain DSM 1800, as described in Example 2 of U.S. Pat. No. 4,810,414 or, the microbial cutinase from *Pseudomonas mendocina* described in U.S. Patent No. 5,512,203, variants thereof and/or equivalents. Suitable variants are described, for example, in WO 03/76580.

Suitable bacterial cutinases may be derived from a *Pseudomonas* or *Acinetobacter* species, preferably from *P. stutzeri, P. alcaligenes, P. pseudoalcaligenes, P. aeruginosa,* or *A. calcoaceticus,* most preferably from *P. stutzeri* strain Thai IV 17-1 (CBS 461.85), PG-1-3 (CBS 137.89), PG-1-4 (CBS 138.89), PG-II-11.1 (CBS 139.89) or PG-II-11.2 (CBS 140.89), *P*. *aeruginosa* PAO (ATCC 15692), *P. alcaligenes* DSM 50342, *P. pseudoalcaligenes* IN II-5 (CBS 468.85), *P. pseudoalcaligenes* M-1 (CBS 473.85), or *A. calcoaceticus* Gr V-39 (CBS 460.85). With respect to the use of cutinases derived from plants, it is known that cutinases exist in the pollen of many plants. Thus, plant-derived cutinases are contemplated for use in the disclosed methods and compositions. Cutinases may also be derived a fungus, such as *Absidia spp.; Acremonium spp.; Agaricus spp.; Anaeromyces spp.; Aspergillus spp*., including *A. auculeatus, A. awamori, A. flavus, A. foetidus, A. fumaricus, A. fumigatus, A. nidulans, A. niger, A. oryzae, A. terreus,* and *A. versicolor; Aeurobasidium spp.; Cephalosporum spp.; Chaetomium spp.; Coprinus spp.; Dactyllum spp.; Fusarium spp*., including *F*. *conglomerans, F. decemcellulare, F. javanicum*, *F. lini, F. oxysporum*, and *F. solani; Gliocladium spp.; Humicola spp*., including *H. insolens* and *H. lanuginosa; Mucor spp.; Neurospora spp*., including *N. crassa* and *N. sitophila; Neocallimastix spp.; Orpinomyces spp.; Penicillium spp; Phanerochaete spp.; Phlebia spp.; Piromyces spp.; Pseudomonas spp.; Rhizopus spp.; Schizophylium spp.; Trametes spp.; Trichoderma spp.,* including *T. reesei, T. reesei (longibrachiatum),* and *T. viride;* and *Zygorhynchus spp.* Similarly, it is envisioned that a cutinase may be found in bacteria such as *Bacillus spp.; Cellulomonas spp.; Clostridium spp.; Myceliophlhora spp.; Pseudomonas spp*., *including P. mendocina and P. putida; Thermomonospora spp.; Thermomyces spp.,* including *T lanuginose; Streptomyces spp., including S. olivochromogenes;* and in fiber degrading ruminal bacteria such as *Fibrobacter succinogenes;* and in yeast including *Candida spp*., *including C. Antarctica, C. rugosa, C. torresii; C. parapsllosis; C. sake; C. zeylanoides; Pichia minuta; Rhodotorula glutinis; R. mucilaginosa;* and *Sporobolomyces holsaticus.*

Cutinases are preferably incorporated in the aqueous enzyme solution in an amount from 0.00001% to 2% of enzyme protein by weight of the fabric, preferably in an amount from 0.0001% to 1% of enzyme protein by weight of the fabric, more preferably in an amount from 0.005% to 0.5% of enzyme protein by weight of the fabric, and even more preferably in an amount from 0.001% to 0.5% of enzyme protein by weight of the fabric.

### 3.2.3 Cellulases

Cellulases are also contemplated for use in the methods and compositions in combination with the *B. subtilis* pectate lyase and variants thereof described herein for bioscouring. Cellulases are classified in a series of enzyme families encompassing endo- and exo- activities, as well as cellobiose hydrolyzing capability. The cellulase(s) used in compositions and processes may be derived from microorganisms which are known to be capable of producing cellulolytic enzymes, such as, *e.g.,* species of *Humicola, Thermomyces, Bacillus, Trichoderma, Fusarium, Myceliophthora, Phanerochaete, Irpex, Scytalidium, Schizophyllum, Penicillium, Aspergillus,* or *Geotricum.* Known species capable for producing celluloytic enzymes include *Humicola insolens, Fusarium oxysporum*, or *Trichoderma reesei*. Non-limiting examples of suitable cellulases are disclosed in U.S. Pat. No. 4,435,307; European patent application No. 0 495 257; PCT Patent Application No. WO 91/17244; and European Patent Application No. EP-A2-271 004.

Cellulases are also useful for biopolishing of a textile. Cotton and other natural fibers (*e.g*., linen, hemp) based on cellulose can be improved by an enzymatic treatment known as "biopolishing." This treatment gives the fabric a smoother and glossier appearance. The treatment is used to remove "fuzz" - the tiny strands of fiber that protrude from the surface of yarn. A ball of fuzz is called a "pill" in the textile trade. After biopolishing, the fuzz and pilling are reduced. The other benefits of removing fuzz are a softer and smoother handle and superior color brightness.

In one embodiment, the cellulase may be used in a concentration in the range from 0.0001% to 1% enzyme protein by weight of the fabric, preferably 0.0001% to 0.05% enzyme protein by weight of the fabric, especially 0.0001 to about 0.01% enzyme protein by weight of the fabric.

Determination of cellulase activity (ECU) may be determined in endo-cellulase units (ECU) by measuring the ability of the enzyme to reduce the viscosity of a solution of carboxymethyl cellulose (CMC), The ECU assay quantifies the amount of catalytic activity present in the sample by measuring the ability of the sample to reduce the viscosity of a solution of carboxymethyl cellulose (CMC). The assay is carried out in a vibration viscosimeter (*e.g*., MIVI 3000 from Sofraser, France) at 40°C; pH 7.5; 0.1 M phosphate buffer; Time = 30 min. using a relative enzyme standard for reducing the viscosity of the CHIC substrate (Hercules 7 LED), enzyme concentration approximately 0.15 ECU/mL). The arch standard is defined to 8200 ECU/g.

One ECU is amount of enzyme that reduces the viscosity to one half under these conditions.

### 3.2.4 Other Bioscouring Enzymes

The compositions and methods using the compositions also contemplated the use of the bioscouring enzymes discussed above in combination with the *B. subtilis* pectate lyase and variants thereof disclosed herein for bioscouring. Other enzymes may be used either alone or in combination with each other or with those listed above. For example, proteases may be used as bioscouring agents. Suitable proteases include those of animal, vegetable or microbial origin, preferably of microbial origin. The protease may be a serine protease or a metalloprotease, preferably an alkaline microbial protease or a trypsin-like protease. Examples of proteases include aminopeptidases, including prolyl aminopeptidase (3.4.11.5), X-pro aminopeptidase (3.4.11.9), bacterial leucyl aminopeptidase (3.4.11.10), thermophilic aminopeptidase (3.4.11.12), lysyl aminopeptidase (3.4.11.15), tryptophanyl aminopeptidase (3.4.11.17), and methionyl aminopeptidase (3.4.11.18); serine endopeptidases, including chymotrypsin (3.4.21.1), trypsin (3.4.21.4), cucumisin (3.4.21.25), brachyurin (3.4.21.32), cerevisin (3.4.21.48)and subtilisin (3.4.21.62); cysteine endopeptidases, including papain (3.4.22.2), ficain (3.4.22.3), chymopapain (3.4.22.6), asclepain (3.4.22.7), actinidain (3.4.22.14), caricain (3.4.22.30), and ananain (3.4.22.31); aspartic endopeptidases, including pepsin A (3.4.23.1), Aspergillopepsin I (3.4.23.18), Penicillopepsin (3.4.23.20), and Saccharopepsin (3.4.23.25); and metalloendopeptidases, including Bacillolysin (3.4.24.28).

Non-limiting examples of subtilisins include subtilisin BPN', subtilisin amylosacchariticus, subtilisin 168, subtilisin mesentericopeptidase, subtilisin Carlsberg, subtilisin DY, subtilisin 309, subtilisin 147, thermitase, aqualysin, Bacillus PB92 protease, proteinase K, protease TW7, and protease TW3.

Commercially available proteases include Alcalase^{™}, Savinase^{™}, Primase^{™}, Duralase^{™}, Esperase^{™}, Kannase^{™}, and Durazym^{™} (Novo Nordisk A/S), Maxatase^{™}, Maxacal^{™}, Maxapem^{™}, Properase^{™}, Purafect^{™}, Purafect OxP^{™}, FN2^{™} and FN3^{™} (Genencor International Inc.).

Additional proteases contemplated for use in the disclosed compositions include variants, such as those disclosed in patents or published patent applications EP 130,756 (Genentech), EP 214,435 (Henkel), WO 87/04461 (Amgen), WO 87/05050 (Genex), EP 251,446 (Genencor), EP 260,105 (Genencor), WO 88/08028 (Genex), WO 88/08033 (Amgen), WO 89/06279 (Novo Nordisk A/S), WO 91/00345 (Novo Nordisk A/S), EP 525 610 (Solvay), WO 94/02618 (Gist-Brocades N.V.), Thomas, et al., 318 NATURE 375-376 (1985), Russel, et al., 328 NATURE 496-500 (1987), Thomas, et al., 193 J. MOL. BIOL. 803-813 (1987).

The activity of proteases can be determined as described in METHODS OF ENZYMATIC ANALYSIS vol. 5 (3rd ed., Verlag Chemie, Weinheim, 1985).

Another aspect contemplates the use of lipases in combination with the disclosed pectate lyase and its variants for bioscouring. Suitable lipases (also, termed carboxylic ester hydrolases) include, without limitation, those of bacterial or fungal origin, including triacylglycerol lipases (3.1.1.3) and Phospholipase A₂ (3.1.1.4.). Contemplated lipases include, without limitation, lipases from *Humicola* (synonym *Thermomyces*), such as from *H. lanuginosa* (*T. lanuginosus*) as described in patents or published patent applications EP 258,068 and EP 305,216, or from *H. insolens* as described in WO 96/13580; a *Pseudomonas* lipase, such as from *P. alcaligenes* or *P. pseudoalcaligenes* (EP 218,272), *P. cepacia* (EP 331,376), *P. stutzeri* (GB 1,372,034), *P. fluorescens, Pseudomonas* sp. strain SD 705 (WO 95/06720 and WO 96/27002), *P. wisconsinensis* (WO 96/12012); a *Bacillus* lipase, such as from *B. subtilis* (Dartois, et al., 1131 BIOCHEM. BIOPHYS. ACTA 253-360 (1993)); *B. stearothermophilus* (JP 64/744992) or *B. pumilus* (WO 91/16422). Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079, and WO 97/07202. Preferred commercially available lipase enzymes include Lipolase^{™}, Lipolase Ultra^{™}, Lipozyme^{™}, Palatase^{™}, Novozym^{™} 435, and Lecitase^{™} (all available from Novo Nordisk A/S). The activity of the lipase can be determined as described in METHODS OF ENZYMATIC ANALYSIS vol. 4 (3rd ed. Verlag Chemie, Weinhein, 1984).

It will be understood that any enzyme exhibiting bioscouring activity can be used in the compositions disclosed. That is, bioscouring enzymes derived from other organisms, or bioscouring enzymes derived from the enzymes listed above in which one or more amino acids have been added, deleted, or substituted, including hybrid polypeptides, may be used, so long as the resulting polypeptides exhibit bioscouring activity. Variants can be created using conventional mutagenesis procedures and identified using, *e.g*., high-throughput screening techniques such as the agar plate screening procedure.

### 3.3 Chemical Bleaching Agents and Enzymatic Bleaching Systems

Another aspect contemplates the use of bleaching agents in combination with *B. subtilis* pectate lyase and variants thereof to treat textiles. No limit is placed on the bleaching agent(s) or systems, as long as the agents and systems do not inactivate the enzymes of the composition. Exemplary bleaching agents include, for example, hydrogen peroxide, carbamide peroxide, sodium carbonate peroxide, sodium peroxide, sodium perborate, sodium hypochlorite, calcium hypochlorite, permanganate, and sodium dichloroisocyanurate. In a preferred embodiment, hydrogen peroxide is used as a bleaching agent. In another embodiment, enzymatic biobleaching agents are used alone or with non-enzymatic bleaching agents. Non-limiting examples of enzymatic biobleaching agents are peroxidases (Colonna, et al., "Recent biological developments in the use of peroxidases," 17 TIBTECH 163-168 (1999)) and oxidoreductases (*e.g*., glucose oxidases) (Pramod, "*Liquid laundry detergents containing stabilized glucose-glucose oxidative system for hydrogen peroxide generation*," U.S. Pat. No. 5,288,746). Bleaching compounds can be present in an aqueous solution in an amount from about 0.01 to about 10 g/L of the aqueous solution or wash liquor, and more preferably from about 0.1 to 5 g/L, and more preferably from about 0.5 to about 2.5 g/L.

Another aspect contemplates the use of perhydrolases in combination with additional chemical bleaching agent(s) such as sodium percarbonate, sodium perborate, sodium sulfate/hydrogen peroxide adduct and sodium chloride/hydrogen peroxide adduct and/or a photosensitive bleaching dye, such as zinc or aluminum salt of sulfonated phthalocyanine further improves the bleaching effects. In additional embodiments, the perhydrolases can be added in further combination with bleach boosters (*e.g*., TAED, NOBS). Bleach boosters can also be utilized in the absence of perhydrolases, but in combination with the disclosed pectate lyases and bleaching agents.

### 3.3.1 Enzymatic Bleaching Systems

Key components to peracid production by enzymatic perhydrolysis are enzyme, ester substrate, and hydrogen peroxide.

### 3.3.1.1. Hydrogen Peroxide

Hydrogen peroxide can be either added directly in batch, or generated continuously *in situ.* The acyl transferase enzymes described in, for example, PCT/US2004/040438, entitled "Perhydrolase", filed December 3, 2004, also find use with any other suitable source of H₂O₂, including that generated by chemical, electro-chemical, and/or enzymatic means. Examples of chemical sources are the percarbonates and perborates, while an example of an electrochemical source is a fuel cell fed oxygen and hydrogen gas, and an enzymatic example includes production of H₂O₂ from the reaction of glucose with glucose oxidase. The following equation provides an example of a coupled system that can be utilized:

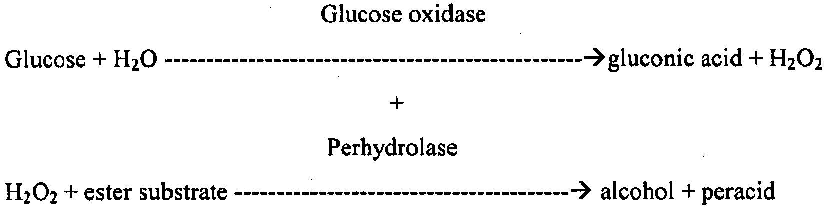

Other enzymes that generate H₂O₂ with a suitable substrate can be used in combination with the compositions and methods described herein. For example, lactate oxidases from *Lactobacillus* species that are known to create H₂O₂ from lactic acid and oxygen. Indeed, one advantage of the methods is that the generation of acid (*e.g*., gluconic acid in the above example) reduces the pH of a basic solution to the pH range in which the peracid is most effective in bleaching (*i.e*., at or below the pKa).

Other enzymes (*e.g*., alcohol oxidase, ethylene glycol oxidase, glycerol oxidase, amino acid oxidase, etc.) that can generate hydrogen peroxide also find use with ester substrates in combination with perhydrolase enzymes to generate peracids. In some preferred embodiments, the ester substrates are selected from one or more of the following acids: formic acid, acetic acid, propionic acid, butyric acid, valeric acid, caproic acid, caprylic acid, nonanoic acid, decanoic acid, dodecanoic acid, myristic acid, palmitic acid, stearic acid, and oleic acid. Thus, as described herein, the compositions and processes described provide definite advantages over the currently used methods and compositions for textile bleaching.

### 3.3.2 Bleach Activators

Any suitable bleach activator may be employed in the present invention. The bleach activators preferred for use in accordance with the invention, include, for example, compounds of the following classes of substances: polyacylated sugars or sugar derivatives with C₁₋₁₀-acyl radicals, preferably acetyl, propionyl, octanoyl, nonanoyl or benzoyl radicals, particularly preferably acetyl radicals, can be used as bleach activators. Sugars or sugar derivatives which can be used are mono- or disaccharides and their reduced or oxidized derivatives, preferably glucose, mannose, fructose, sucrose, xylose or lactose. Particularly suitable bleach activators of this class of substances are, for example, pentaacetylglucose, xylose tetraacetate, 1-benzoyl-2,3,4,6-tetraacetylglucose, and 1-octanoyl-2,3,4,6-tetraacetylglucose.

Another class of substances which are preferred for use as bleach activators include for example acyloxybenzenesulfonic acids and their alkali metal and alkaline earth metal salts, such as C₁₋₁₄-acyl radicals. Acetyl, propionyl, octanoyl, nonanoyl, and benzoyl radicals are preferred, especially acetyl radicals and nonanoyl radicals. Particularly suitable bleach activators in this class of substances are acetyloxybenzenesulfonic acid and benzoyloxybenzenesulfonic acid. They are preferably employed in the form of their sodium salts.

Other bleach activators for use in the present invention include MMA and OCL, alone or in combination with each other or with TAED; O-acyloxime esters, such as acetone O-acetyloxime, acetone O-benzoyloxime, bis(propylimino) carbonate, bis(cyclohexylimino) carbonate as a bleach activator. Acylated oximes which can be used as a bleach activator according to the invention are described, for example, in EP-A-0 028 432. Oxime esters which can be used as a bleach activator according to the invention are described, for example in EP-A-0 267 046.

Additional preferred bleach activators include N-acylcaprolactams, such as N-acetylcaprolactam, N-benzoylcaprolactam, N-octanoylcaprolactam and carbonylbiscaprolactam; N,N-diacylated and N,N,N',N'-tetraacylated amines, such as N,N,N',N'-tetraacetylmethylenediamine and -ethylenediamine (TAED), N,N-diacetylaniline, N,N-diacetyl-p-toluidine or 1,3-diacylated hydantoins such as 1,3-diacetyl-5,5-dimethylhydantoin; N-alkyl-N-sulfonylcarboxamides, such as N-methyl-N-mesylacetamide or N-methyl-N-mesylbenzamide; N-acylated cyclic hydrazides, acylated triazoles or urazoles, such as monoacetylated maleic hydrazide; O,N,N-trisubstituted hydroxylamines, such as O-benzoyl-N,N-succinyl-hydroxylamine, O-acetyl-N,N-succinylhydroxylamine or O,N,N-triacetylhydroxylamine; N,N'-diacylsulfamides, such as N,N'-dimethyl-N,N'-diacetylsulfamide or N,N'-diethyl-N,N'-dipropionylsulfamide; triacylcyanurates, such as triacetylcyanurate or tribenzoylcyanurate; carboxylic anhydrides, such as benzoic anhydride, m-chlorobenzoic anhydride or phthalic anhydride; 1,3-diacyl-4,5-diacyloxyimidazolines, such as 1,3-diacetyl-4,5-diacetoxyimidazoline; tetraacetylglycoluril and tetrapropionylglycoluril; diacylated 2,5-diketopiperazines, such as 1,4-diacetyl-2,5-diketopiperazine; acylation products of propylenediurea and 2,2-dimethylpropylenediurea, such as tetraacetylpropylenediurea; α-acyloxypolyacylmalonamides, such as α-acetoxy-N,N'-diacetylmalonamide; diacyldioxohexahydro-1,3,5-triazines, such as 1,5-diacetyl-2,4-dioxohexahydro-1,3,5-triazine; 2-alkyl- or 2-aryl-(4H)-3,1-benzoxazin-4-ones as described, for example, in EP-B1-0 332 294 and EP-B 0 502 013, and 2-phenyl-(4H)-3,1-benzoxazin-4-one and 2-methyl-(4H)-3,1-benzoxazin-4-one, cationic nitrites, as described, for example, in EP 303 520 and EP 458 396 A1, such as, methosulfates or tosylates of trimethylammonioacetonitrile, N,N-dimethyl-N-octylammonioacetonitrile, 2-(trimethylammonio)propionitrile, 2-(trimethylammonio)-2-methylpropionitrile. Also suitable are the methosulfates of N-methylpiperazinio-N,N'-diacetonitrile and N-methylmorpholinioacetonitrile (MMA).

Additional bleach activators for use in the present invention include percarbamic acids or diacyl percarbamates and precursors thereof.

Bleach activators are typically added in an amount from about 0.1 to 30 g/l, more preferably 0.5 to 10 g/l.

### 3.3.2. Bleach Stabilizers

In another preferred embodiment of the present invention, the bleaching system additionally contains one or more bleach stabilizers. The bleach stabilizers comprise additives able to adsorb, bind or complex traces of heavy metals. Examples of additives that can be used according to the invention with a bleach-stabilizing action include but are not limited to polyanionic compounds, such as polyphosphates, polycarboxylates, polyhydroxypolycarboxylates, soluble silicates as completely or partially neutralized alkali metal or alkaline earth metal salts, in particular as neutral Na or Mg salts, which are relatively weak bleach stabilizers. Examples of strong bleach stabilizers that can be used according to the invention are complexing agents such as ethylenediaminetetraacetate (EDTA), diethylenetriaminepentaacetic acid (DTPA), nitrilotriacetic acid (NTA), methyl-glycinediacetic acid (MGDA), β-alaninediacetic acid (ADA), ethylenediamine-N,N'-disuccinate (EDDS) and phosphonates such as ethylenediaminetetramethylenephosphonate, diethylenetriaminepentamethylenephosphonate (DTMPA) or hydroxyethylidene-1,1-diphosphonic acid in the form of the acids or as partially or completely neutralized alkali metal salts.

The bleach stabilizer is typically added to the treating composition in an amount from about 0.1 to about 5/g liter of the composition, more preferably from about 0.5 to about 2 g/L, and most preferably about 1 g/L.

The bleach composition according to the invention preferably contains at least one bleach stabilizer, and more preferably, at least one of the above mentioned strong bleach stabilizers. Effective bleaching typically results in one or more of the following properties: (i) a desired whiteness (as determined by Ganz whiteness measurement using, *e.g*., a Macbeth color eye); (ii) a satisfactory uniformity of mote removal (assessed by visual examination); preferably, the whiteness of the fabric is 50 Ganz units or higher, and most preferably, 60 Ganz units or higher.

Accordingly, in a preferred embodiment, the single-bath process comprises an enzyme system, such as, pectate lyase, hydrogen peroxide, a bleach activator (such as, TAED) and a bleach stabilizer.

### 3.4 Dyeing Systems

The pectate lyase compositions can be used with various dyeing systems that are compatible with (i) the conditions used for bioscouring, if bioscouring and dyeing are performed simultaneously, or (ii) the conditions as adjusted subsequent to bioscouring, if dyeing is performed after bioscouring. Such dyeing systems include, without limitation:
(a) conventional dyeing systems, comprising one or more of
   (i) direct dyes, such as, C. I. Direct Red 81; Yellow 11 and 28; Orange 39; Red 76; Blue 78, 86, 106, 107 and 108; Black 22;
   (ii) reactive dyes, such as, *e.g.,* C. I. Reactive Red 1, 3, 6, 17, 120, 194; Blue 4, 19, 171 and 182; Black 5; Violet 5;
   (iii) vat dyes, such as, *e.g.,* C. I. Vat Yellow 28; Orange 11 and 15; Blue 6, 16 and 20; Green 1 and 3, 8; Brown 1; Black 9 and 27;
   (iv) sulfur dyes, such as, *e.g.,* C. I. Sulfur Black 1 and 11; Brown 1; Red 10; and
   (v) azoic dyes, such as, *e.g.,* C. I. Coupling Components 5 and 13 in combination with C. I. Azoic Diazo Components 44 and 45. Such conventional dyeing systems are known in the art and are described, *e.g*., in Shore, ed., CELLULOSIC DYEING (Society of Dyers and Colorists, Alden Press, 1995) and in COLOUR INDEX vol. 1-8 Supplements (Society of Dyers and Colorists and American Association of Textile Chemists and Colorists, 1977-1988).

### 3.5 Auxiliary Components

The compositions may also include various auxiliary components, also referred to herein as auxiliary chemicals. Such components include, but are not limited to, sequestering or chelating agents, wetting agents, emulsifying agents, pH control agents (*e.g*., buffers), bleach catalysts, stabilizing agents, dispersing agents, antifoaming agents, detergents and mixtures thereof. It is understood that such auxiliary components are in addition to the enzymes, bleaching agents, and desizing agents.

### 3.5.1 Wetting Agents

Wetting agents are typically selected from surfactants and in particular nonionic surfactants. When employed wetting agents are typically included at levels of from about 0.1 to about 20 g/L, more preferably from about 0.5 to about 10 g/L, and more preferably 0.5 to about 5 g/L of the bath. Stabilizing agents are employed for a variety of reasons including buffering capacity, sequestering, dispersing and in addition enhancing the performance of the surfactants. Stabilizing agents may slow the rate of peroxide decomposition and combine with or neutralize metal impurities that may catalyze decomposition of peroxide and induce fiber damage. Stabilizing agents are well known with both inorganic or organic species being well known and silicates and organophosphates gaining the broadest acceptance and when present are employed at levels of from about 0.01 to about 30 g/L, more preferably from about 0.01 to about 10 g/L, and most preferably from about 0.01 to about 5 g/L of the bath.

### 3.5.2 Surfactants

Surfactants suitable for use include, without limitation, nonionic (*see*, *e.g*., U.S. Pat. No. 4,565,647); anionic; cationic; and zwitterionic surfactants (*see, e.g*., U.S. Pat. No. 3,929,678); which are typically present at a concentration of between about 0.2% to about 15% by weight, preferably from about 1% to about 10% by weight. Anionic surfactants include, without limitation, linear alkylbenzenesulfonate, α-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid, and soap. Non-ionic surfactants include, without limitation, alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, and N-acyl N-alkyl derivatives of glucosamine ("glucamides"). A preferred surfactant for use in embodiments is a non-ionic surfactant or a non-ionic and anionic blend.

### 3.5.3 Chelating Agents

Chelating agents may also be employed and can be selected from the group consisting of amino carboxylates, amino phosphonates, polyfunctionally-substituted aromatic chelating agents and mixtures therein, all as hereinafter defined.

Amino carboxylates useful as optional chelating agents include ethylenediaminetetracetates, N-hydroxyethylethylenediaminetriacetates, nitrilotriacetates, ethylenediamine tetraproprionates, triethylenetetraaminehexacetates, phosphonates to not contain alkyl or alkenyl groups with more than about 6 carbon atoms.

Polyfunctionally-substituted aromatic chelating agents are also useful in the compositions herein. *See e.g*., U.S. Pat. No. 3,812,044. Preferred compounds of this type in acid form are dihydroxydisulfobenzenes, such as 1,2-dihydroxy-3,5-disulfobenzenediethylenetriaminepentaacetates, and ethanoldiglycines, alkali metal, ammonium, and substituted ammonium salts therein and mixtures therein.

Amino phosphonates are also suitable for use as chelating agents in the disclosed compositions when at least low levels of total phosphorus are permitted.

A preferred biodegradable chelator for use herein is ethylenediamine disuccinate ("EDDS"), especially the [S,S] isomer as described in U.S. Pat. No. 4,704,233 issued to Hartman and Perkins. Other chelators contemplated include EDTA and EGTA.

When present, chelating agents are employed at levels of from about 0.01 to about 10 g/L, more preferably from about 0.1 to about 5 g/L, and most preferably from about 0.2 to about 2 g/L.

### 4. Processes for Bioscouring Alone or in Combination with Bleaching, Desizing, and/or Dyeing

The manner in which the aqueous solution containing the enzyme(s) and bleaching system is contacted with the textile material will depend upon whether the processing regime is continuous, semi-continuous, discontinuous pad-batch, batch (or continuous flow). For example, for continuous or discontinuous pad-batch processing, the aqueous enzyme solution is preferably contained in a saturator bath and is applied continuously to the textile material as it travels through the bath, during which process the textile material typically absorbs the processing liquor at an amount of, for example, 0.5 - 1.5 times its weight. In batch operations, the fabric is exposed to the enzyme solution for a period ranging from about 2 minutes to 24 hours at a liquor-to-fabric ratio of 5:1-50:1. These are general parameters. In some embodiments, the time may be shortened by used of more concentrated solutions of the enzymes and other compounds. One skilled in the art is able to determine the parameters best suited for their individual needs.

The methods disclosed herein may be performed at lower temperatures than traditional scouring, desizing and bleaching techniques. In one embodiment, the methods are conducted at temperatures below 95°C, preferably between about 20°C and 75°C. In a more preferred embodiment, the methods are performed at between about 25°C and 60°C.

The methods may be performed at a pH range closer to neutral than traditional desizing, scouring or bleaching techniques. Although the present methods find use at a pH between about 5 and 11, a pH lower than 9 is preferred. In one embodiment, the pH at which the methods are performed lies between about 6 and 9, and preferably between 6 and 8.

One of ordinary skill in the art will recognize that the process conditions to be used may be selected so as to match a particular equipment or a particular type of process which it is desirable to use. For example, while the textile in need of treatment preferably remains in contact with the treatment solution at a temperature of from about 15°C to about 95°C, preferably from about 15°C to about 65°C, most preferably about 20°C to about 45°C and for a period of time suitable for treating the textile which is at least about 2 minutes to 24 hours, more preferably from about 30 minutes to about 12 hours, preferably from about 30 minutes to about 6 hours and most preferably from about 30 to about 90 minutes. Of course, one of ordinary skill in the art will recognize that the reaction conditions such as time and temperature will vary depending upon the equipment and/or process employed and the fabrics treated.

Preferred examples of process types to be used include but not limited to Jet, Jigger/Winch, Pad-Roll and Pad-Steam types. The combined processes may be carried out as a batch, semi-continuous or continuous process using steam or the principles of cold-bleaching. As an example, the process may comprise the following steps: a) impregnating the fabric in a scouring and bleaching bath as described herein followed by squeezing out excessive liquid so as to maintain the quantity of liquor necessary for the reaction to be carried out (normally between 60% and 120% of the weight of the dry fabric); (b) subjecting the impregnated fabric to steaming so as to bring the fabric to the desired reaction temperature, generally between about 20°C and about 80°C; and (c) holding by rolling up or pleating the cloth in a J-Box, U-Box, carpet machine or the like for a sufficient period of time to allow the scouring and bleaching to occur.

As mentioned elsewhere, desizing may be a desired result. Therefore, for certain types of fabric it may be advantageous and/or necessary to subject the fabric to a desizing treatment in order to obtain a final product of a desired quality. In such cases, the combinations of processes may be utilized on the textiles such as a combined de-sizing, bleaching and scouring process, or combined desizing and bleaching process, or a combined desizing and scouring process.

The methods involve providing a non-finished textile component into the treatment solution as described. The textile component may comprise fibers, yarns, fabrics including wovens, knits, garments and non-wovens. By non-finished, it is intended that the textile component be a material that has not been desized, scoured, bleached, dyed, printed, or otherwise provided a finishing step such as durable press. Of course, one of ordinary skill in the art will recognize that the textiles have not been passed through a garment or other manufacturing process involving cutting and sewing of the material.

The present processes may be employed with any textile material including cellulosics such as cotton, linen, ramie, hemp, rayon, lyocell, cellulose acetate, and cellulose triacetate, and synthetic material including but not limited to polyester, nylon, spandex, lycra, acrylics, and various other natural and synthetic material blends. Natural material may include protein fibers such as wool, silk, cashmere, as well as cellulosics as described herein.

The present process may be employed for bleaching without appreciable fiber or fabric damage to several types of synthetic textiles and their blends, including but not limited to polyester, rayon, acetate, nylon, cotton/polyester, cotton/lycra, etc., which may be susceptible to alkaline hydrolysis and degradation.

The methods can further include steps of singeing and mercerization after the treatment step. While desizing may be employed in a separate step, preferably the desizing step is including in the one step treatment via the inclusion of a desizing enzyme(s) in the treatment bath thereby combining, bleaching, de-sizing and scouring into a single step.

Of course the processes includes in the preferred application a washing step or series of washing steps following the one-step treatment methods provided for herein. Washing of treated textiles is well known and within the level of skill of the artisan. Washing stages will be typically present after each of the desizing, scouring, and bleaching steps when present as well as after the treatment step. Washing would also occur after a dyeing step. In addition, the treatment steps, irrespective of their order and/or combinations, may include a wet-out or pre-wetting step to ensure even or uniform wetting in the textile.

Wettability of the textiles is important to any textile dyeing and/or finishing. Wettability leads to superior penetration of the textile by the dye or finish agents and a superior dye and/or finishing result. Accordingly, the wettability of the textile is an indication of how effective the treatment process has been. Higher wettability means a more effective and superior treatment process, *i.e*., a shorter time period for wetting. Conventional textile peroxygen bleaching has provided acceptable wetting profiles only at temperature in excess of 95°C, while lower temperature bleaching (70°C) results in wettability profiles more than about 40%. One test for absorbency is the AATCC Test Method 79-1995, which can be used to quickly check wetting after the treatment.

Fiber damage based on fluidity is measured via AATCC test method 82-1996 involving the dispersion of the fibers in cupriethylene diamine (CP). A representative sample of fibers of about 1.5 mm is cut and dissolved in CP as defined by the equation CP=120 X (sample weight) X 0.98 in a specimen bottle with several glass balls, placed under nitrogen and dissolved by shaking for approximately 2 hours. Additional CP is added as defined by the equation CP=80X(sample weight)X(0.98) and additional shaking under nitrogen for three hours. The solution is placed under constant stirring to prevent separation of the dispersion. The solution can then measured in a calibrated Oswald Canon Fenske viscometer in a constant temperature bath of 25°C to determine the efflux time. Fluidity is calculated from the formula F=100/ctd, where c=viscometer constant, t=efflux time, and d=density of the solution 1.052.

### 4.1 Industrial Applications

The methods and compositions disclosed have many practical applications in industry, as is contemplated herein, and this description is intended to be exemplary, and non-inclusive.

The present compositions, compounds and methods can be used in the processing of fibers, yarns, fabrics, garments, and non-wovens. Major applications include: the one-step enzymatic processing of textiles involving the scouring and bleaching of textiles. The desizing of the textiles, may also be accomplished simultaneously with, the scouring, bleaching, and the scouring, bleaching, and/or biopolishing.

The given dosage (i.e., levels) of the enzyme components in the composition depends on the specific activity, the process conditions and the desired result. The dosage levels can be determined by one of skill in the art.

The compositions and methods described herein provide effective textile treatments with reduced strength loss compared to traditional chemical based treatments, *e.g*., alkali scouring, bleaching, etc. Without being bound by theory, it is believed that the compositions and methods damage the fibers less and thereby reducing strength loss when compared to conventional chemical treatments. Strength loss may be measured by techniques well known in the art such as ASTM D 5034 (Grab test), ASTM D 5035 (Strip test), ASTM D 3787 (Ball burst test), and/or ASTM D 3786 (Hydraulic bursting strength of knitted goods and non-woven fabrics).

The scouring agents can be used alone, or in combination with other scouring agents, desizing agents, bleaching agents, and dyeing agents and in combinations. To ensure good contact between the enzymes and the textile, wetteners should be utilized. Wetteners can be applied before or at the same time with the enzymes.

Mild and strong chelators can be used, as the bioscouring enzyme disclosed is not calcium dependent. Thus, mild chelators such as, but not limited to, polyphosphonates, gluconic acid and citric acid can be used as well as strong chelators such as EDTA. Use of chelators can vary depending on the needs of other enzymes present in the composition. However, calcium removal aids in wax removal and to avoid bleach damage.

### 4.2 Methods for Single-bath Desizing and Scouring

Described herein are one-step or single bath desizing and scouring that occurs at the same conditions as either desizing or scouring. Textiles are treated with a combination of water, desizing enzymes, and a bioscouring enzyme, preferably in combination with one or more agents such as but not limited to stabilizers, surfactants, wetting agents, dispersing agents, sequestering agents, and emulsifying agents, as well as mixtures thereof. The sized fabric is allowed to stand in the processing liquid for a holding period sufficiently long to accomplish the desizing and scouring. The holding period is dependent on the type of processing regimen and the temperatures used, and can vary from about 15 minutes to about 2 hours as well as up to 24 hours.

Processing can be batch, semi-continuous, or continuous, with the textile being contacted by the liquid processing stream in open width or in rope form. Continuous operations generally use a saturator whereby an approximate equal weight of processing liquid per weight of fabric is applied to the fabric, followed by a heated dwell chamber where the chemical reaction takes place. A washing section then prepares the fabric for the next processing step. In order to ensure high whiteness or good wettability, and thereby dyeing, the desizing and scouring enzymes and other agents must be thoroughly removed from the textile. A continuous process can be carried out at about 15°C to about 95°C, for about 5 minutes to about 1 hour, at a pH of about 5 to about 11.

Batch processes generally take place in one processing bath (*i.e*. single bath), whereby the textile is contacted with approximately 8-15 times its weight of processing liquid. After the reaction period, the processing liquid is drained, the textile is rinsed, and the next step performed.

For discontinuous pad-batch processes, a saturator is used whereby an approximate equal weight of processing liquid per weight of fabric is applied to the fabric, followed by a dwell period, which in the case of cold pad-batch process might be one or more days. For instance, a cold pad-batch process can be carried about between 20-40°C for 8-24 hours or more at pH in the range between around 5 and 11. A pad-batch process can be carried out at about 25-85°C for about 1-6 hours and at a similar pH.

For desizing enzymes, such as an alkaline alpha-amylase (*e.g.*, AQUAZYM™ 120L, Novozymes or Purastar™ OxAm, Genencor) is present in a range of about 180 to 240 KNU/L or about 180 to 240 KNU per kg textile. Other ranges of desizing enzymes include but are not limited to 1-100 KNU/kg textile or 2-20 KNU/kg textile. KNU is the alpha amylase activity and can be calculated as discussed in WO 2006/002034.

Generally, a surfactant is added to the process to enhance solubilization of hydrophobic compounds and/or prevent their redeposition back on the textile. Thus, in this context, a detergent is synonymous with a surfactant. Detergents that can be non-ionic surfactants, an anionic surfactant, a cationic surfactant, an ampholytic surfactant, a zwitterionic surfactant, and a semi-polar surfactant or mixture thereof. Surfactants for combined scouring and desizing are generally present in an amount from about 0.1% to about 60% by weight. Surfactants chosen should be compatible with the enzyme components present in the composition. In liquid and gel compositions, surfactants are formulated in a way that at least does not degrade the stability of the enzymes present. Surfactants can be formulated as taught in the art. *See* for example, International PCT Application WO 20006/002034.

### 4.3 Methods for Combined Bleaching, Scouring, and/or Desizing

In one aspect, combined scouring and bleaching using the disclosed lyase is contemplated. Thus, scouring and bleaching with the enzyme in the presence of an enzymatic bleaching system and a peroxide is contemplated with an optimal pH range of about 6 to about 8 for pectin removal. A preferred enzymatic bleaching system is that of acyl transferase.

For cold pad batch processes, the temperature is preferable between about 15°C and 45°C and more preferably from between about 25°C to about 35°C. Higher temperatures may be used for non-cold pad batch processes of up to 95°C. The aqueous solution or wash liquor typically has a pH of between about 5 and about 11. Preferably, the pH of the treating composition is between about 5 and about 9, and more preferably between about 6 to about 8.

The single-bath method for treating textiles may be carried out without the addition of alkali. Preferably, this treatment is used for treating alkaline-sensitive textiles, such as but not limited to silk, wool, nylon, polyester, lycra, cellulose acetate, and blends. In another embodiment, the single-bath method for treating textiles is carried out at a pH below 9, more preferably, below 8, and even more preferably below 7.

The single-bath method for treating textiles may be carried out with the addition of alkaline. Preferably, the contacting is performed at a pH range of about 8 to about 11. The pH may be controlled, e.g., with the addition of an alkali agent, such as NaOH. Alkali agents may be added in amounts from about 0.1 to about 10% by wt. of fabric, as necessary to obtain the desired pH. However, as an artisan would appreciated, the amount of alkali agent added with depend on the amount of bleaching compound used.

It will be understood that the optimum dosage and concentration of the enzymes, bleaching compounds, bleach stabilizers, and alkali agents (if used), the volume of the aqueous solution or wash liquor, and the pH and temperature will vary, depending on: (i) the nature of the fiber, *i.e.,* crude fiber, yarn, or textile; (ii) whether simultaneous or sequential scouring and bleaching are carried out; (iii) the particular enzyme(s) used, and the specific activity of the enzyme; (iv) the conditions of temperature, pH, time, *etc*., at which the processing occurs; (v) the presence of other components in the wash liquor; and (vi) the type of processing regime used, *i.e*., continuous, discontinuous pad-batch, or batch. The optimization of the process conditions can be determined using routine experimentation, such as, by establishing a matrix of conditions and testing different points in the matrix. For example, the amount of enzyme, the temperature at which the contacting occurs, and the total time of processing can be varied, after which the resulting cellulosic materials or textile is evaluated for (a) pectin removal; (b) a scoured property such as, e.g., wettability; and (c) quality of bleaching, such as whiteness.

The conditions or treating composition may be adjusted to favor the desizing, scouring or bleaching processes, such as, by adjusting pH, concentration of wetting agent, or concentration of divalent cationic chelator such as ethylene diamine tetraacetate so as to further promote the bleaching process. The sequential mode may further comprise adjusting one or more properties of the composition of the aqueous solution or wash liquor between steps (ii) and (iii). For example, pH, concentration of wetting agent, or concentration of divalent cationic chelator, such as, ethylene diamine tetraacetate, may be adjusted between steps (ii) and (iii) so as to further promote the bleaching process. The conditions of the first and second incubations may also differ with respect to temperature, agitation, time, and the like.

### 4.4 Methods for Single-bath Biopreparation and Dyeing

Biopreparation (or scouring) and dyeing may be performed in a single bath. There are at least two modes of practicing the methods. A bioscouring enzyme and a dyeing system may be added to the aqueous solution or wash liquor that contacts the cellulosic fiber or fabric, and incubation is performed for sufficient time and under appropriate conditions to achieve both effective scouring and effective dyeing. In an alternative method, (i) a bioscouring enzyme is added to the wash liquor; (ii) a first incubation is performed for sufficient time and under appropriate conditions to at least initiate, and preferably to achieve, effective scouring; (iii) the wash liquor containing the bioscouring enzyme is then supplemented with a dyeing system; and (iv) a second incubation is performed for a sufficient time and under appropriate conditions to achieve effective dyeing. It will be understood that the second described method may further comprise adjusting one or more properties of the composition of the wash liquor between steps (ii) and (iii) (such as, *e.g.,* pH, ionic strength, concentration or wetting agent, or concentration of divalent cationic chelator such as EDTA or EGTA, and that the conditions of the first and second incubations may also differ with respect to temperature, agitation, pH, time, and the like.

The concentration of enzyme(s) in the aqueous solution can be adjusted so that the dosage of enzyme(s) added to a given amount of fiber is between about 0.1 and about 10,000 mol/min/kg fiber, preferably between about 1 and about 2,000 mol/min/kg fiber, and most preferably between about 10 and about 500 mol/min/kg fiber. Using a different unit of measure, the dosages of enzyme(s), therefore can be for example between about 250 and 12,000 APSU/kg fiber (APSU means alkaline pectinase standard unit), preferably between about 500 and 9000 APSU/kg fiber, and most preferably between about 1000 and 6000 APSU/kg fiber.

The aqueous solution containing the bioscouring enzyme has a pH of between about 5 and about 11, and more preferably from about 6-10. The preferred pH will depend on whether scouring and dyeing are performed simultaneously or sequentially. If scouring and dyeing are performed simultaneously, the wash liquor preferably has a pH of between about 5 and about 8.5, and most preferably between about 7 and about 8. When the steps are performed sequentially, the wash liquor in steps (i) and (ii) may preferably have a pH between about 6 to 9, *e.g*., between about 7.0 and about 8.5, and in steps (iii) and (iv) between about 6 and about 9. Furthermore, the wash liquor preferably either contains a low concentration of added calcium, i.e., less than 2 mM Ca²⁺ or lacks added Ca²⁺ or other divalent cations entirely.

When bioscouring and dyeing simultaneously, the temperature at which the combined scouring and dyeing processes are carried out may be between about 15°C and about 95°C, more preferably between about 20°C and 80°C, and most preferably between about 40°C and 70°C. Similar temperatures can be utilized when the steps of bioscouring and dyeing occur sequentially, or the temperatures can vary to include ranges between about 25°C and about 50°C for the scouring step; and the temperature at which the subsequent dyeing is carried out may be between about 30°C and about 100°C, preferably below 50°C. It will be understood that the choice of temperature(s) will depend on (i) the nature of the fiber, *i.e*., crude fiber, yarn, or textile; (ii) the particular enzyme used for scouring, as well as the particular oxidative enzyme if used for dyeing; and (iii) the particular dye or dye type. For example, exhaust dyeing is normally carried out between about 60°C and 95°C. Direct dyeing using reactive dyeing is carried out for example at 60°C to 80°C and sulfur dyeing occurs around about 95°C. Additionally, both sulfur and direct dyeing (*e.g*., Ciba dyes such as Solophenyl) require a high concentration of salt (*e.g*., NaCl). Reactive and direct dyeing also require a pH preferably in the range of about pH 6 to 8, which works well with the pectate lyase disclosed herein.

Effective scouring typically results in a wettability of less than about 10 seconds, preferably less than about 5 seconds, and most preferably less than about 2 seconds, when measured using the drop test according to AATCC Test Method 39-1980. Typically, effective scouring requires the digestion of a substantial proportion of the pectin in the fiber, preferably at least 30% by weight, more preferably at least 50% by weight, and most preferably at least 70%. Pectin digestion refers to cleavage of α-1,4-glycosidic linkages in pectin so that the digestion products can be removed from the fiber by, *e.g*., rinsing or any other conventional separation method. Methods for measuring the degree of pectin digestion of a fiber include, without limitation, the Ruthenium Red staining method as described by, for example, Luft, 171 THE ANATOMICAL RECORD 347 (1971).

Effective dyeing typically results in one or more of the following properties: (i) a desired color shade and depth (as determined by CIE L*a*b* measurements using, *e.g*., a Mecbeth color eye); (ii) a satisfactory uniformity of dyeing (assessed by visual examination); and (iii) dyeing fastness properties such as washing fastness, light fastness, and crocking (wet and dry) fastness of least about 3.0, preferably above 3.5, and most preferably above 4.0 (as measured on a color gray scale using Method EP1 as disclosed in AATCC TECHNICAL MANUAL, vol. 7, 350 (1995)).

Furthermore, the methods using compositions with a *B. subtilis* pectate lyase may result in enhanced uptake of dye in fibers subjected to single-vat bioscouring and dyeing relative to fibers subjected only to dyeing. Preferably, the enhancement of dye uptake is at least about 10%. Dye uptake may be measured by for example: (i) measuring exhaustion of a dye solution, or (ii) measuring the intensity of color in the fabric (L*a*b* value).

To achieve effective scouring, the dosage of enzyme(s) (mol/min/kg fiber), the concentration of enzyme(s) in the wash liquor (mol/min/L wash liquor), and the total volume of wash liquor applied to a given amount of fiber (L/kg fiber) will vary, depending on: (i) the nature of the fiber, *i.e*., crude fiber, yarn, or textile; (ii) whether simultaneous or sequential scouring and dyeing are carried out; (iii) the particular enzyme(s) used, and the specific activity of the enzyme; (iv) the conditions of temperature, pH, time, etc., at which the processing occurs; and (v) the presence of other components in the wash liquor.

Determination of suitable conditions, including, *e.g.,* enzyme dosage, enzyme concentration (or concentration of enzymes in a mixture of enzymes), volume of solution, and temperature to be used can be achieved using routine experimentation by establishing a matrix of conditions and testing different points in the matrix. For example, the amount of enzyme, the temperature at which the contacting occurs, and the total time of processing can be varied, after which the resulting fiber or textile is evaluated for (a) pectin removal; (b) a scoured property such as, *e.g*., wettability; and (c) quality of dyeing.

In simultaneous scouring and dyeing, the fiber is contacted with pectate lyase (optionally in the presence of other enzymes) and a cellulosic dye such as C.I. Reactive Blue 184 under the following conditions: (i) a temperature of about 45°C; (ii) a pH of about 7.0-8.0; (iii) the absence of added divalent cations; (iv) a wash liquor:fabric ratio of between about 0.5 and about 50; and (v) a bioscouring enzyme dosage of between about 10 and about 500 mol/min/kg fiber.

In the examples below it is shown that the scouring step can be carried out using a disclosed pectate lyase or pectate lyase preparation at a temperature of 50°C or less (but above 0°C) and optimally at room temperature. The pH of the combined bleaching bioscouring reaction can be from about 6 to about 11, and optimally about 6.0 to 8.5.

### 5.1.1 Textile Surfactants

Described herein is an aqueous composition containing *B. subtilis* pectate lyase further comprising a surfactant exhibiting a compatible or synergistic response with the enhanced bleaching and/or scouring effect. The surfactant-fortified compositions can comprise a surfactant system, wherein the surfactant can be selected from honionic and/or anionic and/or cationic and/or ampholytic and/or zwitterionic and/or semi-polar surfactant, as a combination of such surfactants in combination with a pectate lyase. Other enzymes can also be included in the composition.

The surfactant is typically present at a level from 0.1% to 60% (more preferably 0.2% - 15%) by weight and is most preferably formulated in such a way that it promotes, or at least does not degrade, the stability of any enzyme in these compositions.

Preferred systems to be used comprise a non-ionic and/or anionic surfactants. Polyethylene, polypropylene, and polybutylene oxide condensates of alkyl phenols are suitable for use as the nonionic surfactant of surfactant systems, with the polyethylene oxide condensates being preferred. The condensation products of primary and secondary aliphatic alcohols with about 1 to about 25 moles of ethylene oxide are suitable for use as the nonionic surfactant of nonionic surfactant systems. Also useful as a nonionic surfactant are alkylpolysaccharides, as described in for example U.S. Patent No. 4,565,647. The condensation products of ethylene oxide with a hydrophobic base formed by the condensation of propylene oxide with propylene glycol are also suitable for use. Other nonionic surfactants useful in the described compositions include condensation products of ethylene oxide with the product resulting from the reaction of propylene oxide and ethylenediamine, as well as alcohol ethoxylate, nonylphenol ethoxylate, alkylpolyglycoside, alkyldimethylamineoxide, ethoxylated fatty acid monoet-hanolamide, fatty acid monoethanolamide, polyhydroxy alkyl fatty acid amide, and N-acyl N-alkyl derivatives of glucosamine ("glucamides").

Preferred anionic surfactants include alkyl alkoxylated sulfate surfactants and the analogous phosphate esters. Suitable anionic surfactants to be used are alkyl ester sulfonate surfactants, olefinsulfonates, alkyl sulfates (fatty alcohol sulfates), alcohol ethoxy sulfates, secondary alkane sulfonates, alpha-sulfo fatty acid methylesters, alkyl- or alkenylsuccinic acid, and soap, including linear esters of C8-C20 carboxylic acids (*i.e*., fatty acids), which are sulfonated with gaseous SO₃. Other anionic surfactants useful for textile cleaning can also be included in an aqueous enzyme composition. Aqueous enzyme compositions can also contain cationic, ampholytic, zwitterionic, and semi-polar surfactants, as well as the nonionic and/or anionic surfactants other than those already described herein.

When included therein, the aqueous enzyme compositions typically comprise from about 1% to about 40% by weight and more preferably from about 3% to about 20% by weight of surfactants.

### 5.1.2 Antifoaming agents

Another optional ingredient in textile cleaning, bleaching and scouring compositions may be a foam suppressor or antifoaming agent. Exemplary antifoaming agents include silicones, DC-S44 (Dow Coming) and silica-silicone mixtures. The antifoaming agents are normally employed at levels of from 0.001% to 2% by weight of the composition, preferably from 0.01% to 1% by weight. *See, e.g*., U.S. Patent No. 3,933,622.

### 5.1.3 Proteases

Suitable proteases for use in the described compositions include those of animal, vegetable or microbial origin. Preferably, the proteases are of microbial origin. The protease may be a serine protease or a metalloprotease, such as an alkaline microbial protease or a trypsin-like protease. Examples of proteases include but are not limited to
(a) aminopeptidases including but not limited to prolyl aminopeptidase (EC 3.4.11.5), X-pro aminopeptidase (EC 3.4.11.9), bacterial leucyl aminopeptidase (EC 3.4.11.10), thermophillic aminopeptidase (EC 3.4.11.12), lysyl aminopeptidase (EC 3.4.11.15), tryptophanyl aminopeptidase (EC 3.4.11.17), and methionyl aminopeptidase (EC 3.4.11.18);
(b) serine endopeptidases including but not limited to chymotrypsin (EC 3.4.21.1), trypsin (EC 3.4.21.4), cucumisin (EC 3.4.21.25), brachyurin (EC 3.4.21.32), cerevisin (EC 3.4.21.48), and subtilisin (EC 3.4.21.62);
(c) cysteine endopeptidases including but not limited to papain (EC 3.4.22.2), ficain (EC 3.4.22.3), chymopapain (EC 3.4.22.6), asclepain (EC 3.4.22.7), actinidain (EC 3.4.22.14), caricain (EC 3.4.22.30), and ananain (EC 3.4.22.31);
(d) aspartic endopeptidases including but not limited to pepsin A (EC 3.4.23.1), Aspergillopepsin I (EC 3.4.23.18), Penicillopepsin (EC 3.4.23.20), and Saccharopepsin (EC 3.4.23.25); and
(e) metalloendopeptidases such as Bacillolysin (EC 3.4.24.28).

Non-limiting examples of subtilisins include subtilisin BPN', subtilisin amylosacchariticus, subtilisin 168, subtilisin mesentericopeptidase, subtilisin Carlsberg, subtilisin DY, subtilisin 309, subtilisin 147, thermitase, aqualysin, Bacillus PB92 protease, proteinase K, protease TW7, and protease TW3.

Commercially available proteases include Alcalase™, Savinase™, Primase™, Duralase™, Esperase™, Kannase™, and Durazym™ (Novo Nordisk A/S), Maxatase™, Maxacal™, Maxapem™, Properase™, Purafect™, Purafect OxP™, FN2™, and FN3™ (Genencor International Inc.).

Also useful in the compositions and methods are protease variants, such as those disclosed in European (EP) Patent Application Nos. 130756 (Genentech); EP 260105 (Genencor); EP 251446 (Genencor); European Application 525610 (Solvay); and EP 214435 (Henkel); International PCT Application Nos. WO 87/04461 (Amgen); WO 87/05050 (Genex); WO 88/08028 (Genex); WO 88/08033 (Amgen); WO 89/06279 (Novo Nordisk A/S); WO 91/00345 (Novo Nordisk A/S); and WO 94/02618 (Gist-Brocades N.V.); and Thomas et al., 318 NATURE 375-376 (1985); Thomas et al., 193 J. MOL. BIOL. 193: 803-813 (1987); Russel et al., 328 NATURE 496-500 (1987).

Activity of proteases can be determined for example as described in METHODS OF ENZYMATIC ANALYSIS, vol. 5 (3rd ed. Verlag Chemie, Weinheim 1984).

### 5.1.4 Lipases

Lipases are also contemplated for inclusion in a composition comprising *B. subtilis* pectate lyase. Suitable lipases (also termed carboxylic ester hydrolases) include, without limitation, those of bacterial or fungal origin, including triacylglycerol lipases (EC 3.1.1.3) and Phospholipase A₂ (EC 3.1.1.4). Additional lipases include but are not limited to those from *Humicola* (synonym *Thermomyces*), such as from *H. lanuginosa* (*T. lanuginosus*) as described in for example European Patent Nos. 258 068 and 305 216, or from *H. insolens* as described in International PCT Application WO 96/13580; a *Pseudomonas* lipase, such as from *P. alcaligenes* or *P. pseudoalcaligenes* (EP Patent No. 218 272), *P. cepacia* (EP Patent No. 331 376), *P. stutzeri* (Great Britain Patent No. 1,372,034), *P. fluorescens, Pseudomonas* sp. strain SD 705 (*see e.g*., International PCT Application Nos. WO 95/06720 and WO 96/27002), *P. wisconsinensis* (International PCT Application No. WO 96/12012); a *Bacillus* lipase, such as from *B. subtilis* (*see e.g*., Dartois et al., 1131 BIOCHEM. BIOPHYS. ACTA 253-360 (1993)); *B. stearothermophilus* (Japanese Application No. 64/744992) or *B. pumilus* (International PCT Application No. WO 91/16422). Other examples may include lipase variants such as those described in International PCT Application Nos. WO 92/05249, WO 94/01541, WO 95/35381, WO 96/00292, WO 95/30744, WO 94/25578, WO 95/14783, WO 95/22615, WO 97/04079, and WO 97/07202; and European Patent Nos. 407 225 and 260 105. Commercially available lipase enzymes include, but are not limited to Lipolase™, Lipolase Ultra™, Lipozyme™, Palatase™, Novozym™ 435, and Lecitase™ (all available from Novo Nordisk A/S). The activity of a lipase in a composition can be determined, for example, as described in METHODS OF ENZYMATIC ANALYSIS, vol. 4 (3rd ed., Verlag Chemie, Weinheim, 1984).

Additional bioscouring enzymes can be used with the pectate lyase. Such additional bioscouring enzymes can be derived from other microorganisms, or bioscouring enzymes derived from the enzymes listed above in which one or more amino acids have been added, deleted, or substituted, including hybrid polypeptides. Such derivative and hybrid polypeptides may be used so long as the resulting polypeptides exhibit bioscouring activity. Such variants can be created using conventional mutagenesis procedures and identified using, *e.g*., high-throughput screening techniques such as the agar plate screening procedures. For example, pectate lyase activity may be measured by applying a test solution to 4 mm holes punched out in agar plates (*e.g*., LB agar), containing 0.7% w/v sodium polygalacturonate (Sigma P 1879). The plates are then incubated for 6 h at a particular temperature (*e.g*., 25-40°C). The plates are then soaked in for example either (i) 1 M CaCl₂ for 0.5 h; or (ii) 1% mixed alkyl trimethylammonium bromide (MTAB, Sigma M-7635) for 1 hour. Both of these procedures cause the precipitation of polygalacturonate within the agar. Pectate lyase activity can be detected by the appearance of clear zones within a background of precipitated polygalacturonate. Sensitivity of the assay is calibrated using dilutions of a standard preparation of pectate lyase.

Determination of temperature, pH, and divalent cation dependence of an isolated bioscouring enzyme can be achieved using conventional methods. For example, an enzymatic activity assay may be performed at a range of temperatures and pHs, and in the presence and absence of different concentrations of Ca⁺², and the temperature and pH optima and divalent cation effect (if any) are quantified. Temperature, pH, and cation dependence are then determined to establish the suitability of a particular pectate lyase for use in the compositions and methods of use presently contemplates. These can be tested across an array of substrates as well.

Bioscouring enzymes may be derived from their cell of origin or may be recombinantly produced, and may be purified or isolated. As used herein, a "purified" or "isolated" enzyme is one that has been treated to remove non-enzyme material derived from the cell in which it was synthesized that could interfere with its enzymatic activity. Typically, the bioscouring enzyme is separated from the bacterial or fungal microorganism in which it is produced as an endogenous constituent or as a recombinant product. If the enzyme is secreted into the culture medium, purification may comprise separating the culture medium from the biomass by centrifugation, filtration, or precipitation, using conventional methods. Alternatively, the enzyme may be released from the host cell via cell disruption, and then separated from the biomass. In some cases, further purification may be achieved by conventional protein purification methods, including without limitation ammonium sulfate precipitation; acid or chaotrope extraction; ion exchange; molecular sieve; and hydrophobic chromatography, such as FPLC and HPLC; preparative isoelectric focusing; and preparative polyacrylamide gel electrophoresis. Alternatively, purification may be achieved using affinity chromatography such as immunoaffinity chromatography. For example, hybrid recombinant pectate lyases may be used having an additional amino acid sequence that serves as an affinity "tag", which facilitates purification using an appropriate solid-phase matrix.

The bioscouring enzyme may be chemically modified to enhance one or more properties that provide advantageous characteristics, such as *e.g.,* increasing solubility, decreasing lability, or divalent ion dependence, etc. The modifications include, without limitation, phosphorylation, acetylation, sulfation, acylation, or other protein modifications known to those skilled in the art.

### 5.1.5 Builder Systems

The compositions described herein may further comprise a builder system. Any conventional builder system is suitable for use herein including aluminosilicate materials, silicates, polycarboxylates and fatty acids, materials such as ethylenediamine tetraacetate, metal ion sequestrants such as aminopolyphosphonates, particularly ethylenediamine tetramethylene phosphonic acid and diethylene triamine pentamethylenephosphonic acid. Though less preferred for obvious environmental reasons, phosphate builders can also be used herein.

Suitable builders can be an inorganic ion exchange material, commonly an inorganic hydrated aluminosilicate material, more particularly a hydrated synthetic zeolite such as hydrated zeolite A, X, B, HS or MAP.

Another suitable inorganic builder material is layered silicate, *e.g.* SKS-6 (Hoechst). SKS-6 is a crystalline layered silicate consisting of sodium silicate (Na₂Si₂O₅).

Suitable polycarboxylates containing one carboxy group include lactic acid, glycolic acid and ether derivatives thereof. Polycarboxylates containing two carboxy groups include the water-soluble salts of succinic acid, malonic acid, (ethylenedioxy) diacetic acid, maleic acid, diglycollic acid, tartaric acid, tartronic acid and fumaric acid, as well as the ether carboxylates described in for example U.S. Pat. No. 3,935,257 and the sulfinyl carboxylates. Polycarboxylates containing three carboxy groups include, in particular, water-soluble citrates, aconitrates and citraconates as well as succinate derivatives such as the carboxymethyloxysuccinates, lactoxysuccinates, and the oxypolycarboxylate materials such as 2-oxa-1,1,3-propane tricarboxylates.

Polycarboxylates containing four carboxy groups include oxydisuccinates; 1,1,2,2,-ethane tetracarboxylates; 1,1,3,3-propane tetracarboxylates containing sulfo substituents (*see, e.g.,* U.S. Pat. No. 3,936,448); and the sulfonated pyrolysed citrates; and polycarboxylates containing phosphone substituents.

Alicyclic and heterocyclic polycarboxylates include cyclopentane-cis,cis-cistetracarboxylates; cyclopentadienide pentacarboxylates; 2,3,4,5-tetrahydro-furan-cis,cis; cistetracarboxylates; 2,5-tetrahydro-furan-cis; discarboxylates; 2,2,5,5,-tetrahydrofuran tetracarboxylates; 1,2,3,4,5,6-hexane-hexacarboxylates and carboxymethyl derivatives of polyhydric alcohols such as sorbitol, mannitol and xylitol. Aromatic polycarboxylates include mellitic acid, pyromellitic acid and the phthalic acid derivatives disclosed.

Preferred polycarboxylates include hydroxy-carboxylates containing up to three carboxy groups per molecule, more particularly citrates.

Preferred builder systems for use in the present compositions include a mixture of a water-insoluble aluminosilicate builder such as zeolite A or of a layered silicate (SKS-6), and a water-soluble carboxylate chelating agent such as citric acid.

A suitable chelant for inclusion in the detergent compositions in accordance with the invention is ethylenediamine-N,N'-disuccinic acid (EDDS) or the alkali metal, alkaline earth metal, ammonium, or substituted ammonium salts thereof, or mixtures thereof. Preferred EDDS compounds are the free acid form and the sodium or magnesium salt thereof. Examples of such preferred sodium salts of EDDS include Na₂EDDS and Na₄EDDS. Examples of such preferred magnesium salts of EDDS include MgEDDS and Mg₂EDDS. The magnesium salts are the most preferred for inclusion in compositions in accordance with the invention.

Also contemplated are builder materials that can form part of the builder system for use in granular compositions including but not limited to inorganic materials such as alkali metal carbonates, bicarbonates, silicates, and organic materials such as the organic phosphonates, amino polyalkylene phosphonates and amino polycarboxylates.

Other suitable water-soluble organic salts are the homo- or co-polymeric acids or their salts, in which the polycarboxylic acid comprises at least two carboxyl radicals separated form each other by not more than two carbon atoms. Polyacrylate salts include those of a molecular weight of 2000 to 5000 Daltons and their copolymers with maleic anhydride, such copolymers having a molecular weight of from 20,000 to 70,000, especially about 40,000.

Detergency builder salts are normally included in amounts of from 5% to 80% by weight of the composition. Preferred levels of builder for liquid detergents are from 5% to 30%.

### 5.1.6 Bleaching Agents

Additional optional detergent ingredients that can be included in the detergent compositions described herein include bleaching agents such as perborate PB1, PB4 and percarbonate. These bleaching agent components can include one or more oxygen bleaching agents and, depending upon the bleaching agent chosen, one or more bleach activators. Present oxygen bleaching compounds will typically be present at levels of from about 1% to about 25%. In general, bleaching compounds are optional added components in non-liquid formulations, e.g. granular detergents. It can also include bleaching systems as discussed herein.

The bleaching agent component for use herein can be any of the bleaching agents useful for detergent compositions including oxygen bleaches as well as others known in the art.

The bleaching agent suitable for the present invention can be an activated or nonactivated bleaching agent.

One category of oxygen bleaching agent that can be used encompasses percarboxylic acid bleaching agents and salts thereof. Suitable examples of this class of agents include magnesium monoperoxyphthalate hexahydrate, the magnesium salt of meta-chloro perbenzoic acid, 4-nonylamino-4-oxoperoxybutyric acid and diperoxydodecanedioic acid. Such bleaching agents are disclosed in U.S. Pat. No. 4,483,781, EP 0 133 354, and U.S. Patent No. 4,412,934. Highly preferred bleaching agents also include 6-nonylamino-6-oxoperoxycaproic acid such as those described in U.S. Patent No. 4,634,551.

Another category of bleaching agents encompasses the halogen bleaching agents. Examples of hypohalite bleaching agents, for example, include trichloro isocyanuric acid and the sodium and potassium dichloroisocyanurates and N-chloro and N-bromo alkane sulphonamides. Such materials are normally added at 0.5-10% by weight of the finished product, preferably 1-5% by weight. Such halogen bleaching agents are generally less preferred for use in enzymatic detergents.

The hydrogen peroxide releasing agents can be used in combination with bleach activators such as tetra-acetylethylenediamine (TAED), nonanoyloxybenzenesulfonate (NOBS, *see, e.g*., U.S. Pat. No. 4,412,934), 3,5-trimethyl-hexsanoloxybenzenesulfonate (ISONOBS, *see e.g.,* EP 120 591) or pentaacetylglucose (PAG), which are perhydrolyzed to form a peracid as the active bleaching species, leading to improved bleaching effect. In addition, very suitable are the bleach activators C8 (6-octanamido-caproyl) oxybenzene-sulfonate, C9(6-nonanamido caproyl) oxybenzenesulfonate, and C10 (6-decanamido caproyl) oxybenzenesulfonate or mixtures thereof. Also suitable activators are acylated citrate esters such as disclosed in European Patent Application No. 91870207.7.

Useful bleaching agents, including peroxyacids and bleaching systems comprising bleach activators and peroxygen bleaching compounds for use in cleaning compositions.

The hydrogen peroxide may also be present by adding an enzymatic system (i.e. an enzyme and a substrate therefore) which is capable of generation of hydrogen peroxide at the beginning or during the washing and/or rinsing process. Such enzymatic systems are disclosed in European Patent Application EP 0 537 381.

Bleaching agents other than oxygen bleaching agents are also known in the art and can be utilized herein. One type of non-oxygen bleaching agent of particular interest includes photoactivated bleaching agents such as the sulfonated zinc and/or aluminium phthalocyanines. These materials can be deposited upon the substrate during the washing process. Upon irradiation with light, in the presence of oxygen, such as by hanging clothes out to dry in the daylight, the sulfonated zinc phthalocyanine is activated and, consequently, the substrate is bleached. Preferred zinc phthalocyanine and a photoactivated bleaching process are described in for example U.S. Patent No. 4,033,718. Typically, a detergent composition will contain about 0.025% to about 1.25%, by weight, of sulfonated zinc phthalocyanine.

Bleaching agents may also comprise a manganese catalyst. The manganese catalyst may, e.g., be one of the compounds described in "Efficient manganese catalysts for low-temperature bleaching," 369 NATURE 637-639 (1994).

### 5.1.7 Other components

Additional components that can be used in the detergent compositions in various combinations include soil-suspending or anti-redeposition agents, soil-releasing agents, optical brighteners, abrasives, bactericides, tarnish inhibitors, coloring agents, and/or encapsulated or nonencapsulated perfumes.

Especially suitable encapsulating materials are water soluble capsules which consist of a matrix of polysaccharide and polyhydroxy compounds.

Other suitable water soluble encapsulating materials comprise dextrins derived from ungelatinized starch acid esters of substituted dicarboxylic acids (*see*, *e.g*., U.S. Patent No. 3,455,838). Such acid-ester dextrins are, preferably, prepared from such starches as waxy maize, waxy sorghum, sago, tapioca and potato. Suitable examples of said encapsulation materials include N-Lok manufactured by National Starch. The N-Lok encapsulating material consists of a modified maize starch and glucose. The starch is modified by adding monofunctional substituted groups such as octenyl succinic acid anhydride.

Typical anti-redeposition agents used in detergents include water-soluble, generally organic colloids, including for example the water-soluble salts of polymeric carboxylic acids such as polyacrylic acid or polymaleic acid or co-polymers thereof, glue, gelatine, salts of ether carboxylic acids or ether sulfonic acids of starch or cellulose or salts of sulfuric acid esters of cellulose or starch. Water-soluble polyamides containing acidic groups are also used as anti-redeposition agent. Soluble starch preparations and other starch products than those mentioned above, for example partly hydrolyzed starch, may also be used. Sodium carboxymethyl cellulose, methyl cellulose, hydroxyethyl cellulose, methyl hydroxyethyl cellulose and mixtures thereof are preferably used. These materials are normally used at levels of from 0.05% to 10% by weight, more preferably form 0.2% to 8%, most preferably from 0.5% to 6% by weight of the composition.

Also contemplated are the use of optical brighteners. Preferred optical brighteners are anionic in character, examples of which are disodium 4,4'-bis-(2-diethanolamino-4-anilino-s-triazin-6-ylami- no)stilbene-2:2' disulpho-nate; disodium 4,4'-bis-(2-morpholino-4-anilino-s-triazin-6-ylamino-stilbene-2:2'-disulphonate; disodium 4,4'-bis-(2,4-dianilino-s-triazin-6-ylamino)stilbene-2,2'-disulphonate; monosodium 4',4"-bis-(2,4-dianilino-s-tri-azin-6-ylamino)stilbene-2-sulphonate; disodium 4,4'-bis-(2-anilino-4-(N-methyl-N-2-hydroxyethylamino)-s-triazin-6-ylamino)-stilbene-2,2'-disulphonate; disodium 4,4'-bis-(4-phenyl-2,1,3-triazot-2-yl)-stilbene-2,2' disulphonate; disodium 4,4'bis(2-anilino4-(1-methyl-2-hydroxyethylamino)-striazin-6-yla- mi-no)stilbene-2,2'disulphonate; sodium 2(stilbyl-4"-(naphtho-1',2':4,5)-1,2,3,-triazole-2"-sulphonate; and 4,4'-bis(2-sulphostyryl)biphenyl.

Other useful polymeric materials are the polyethylene glycols, particularly those of molecular weight about 1000-10000 Daltons, more particularly about 2000 to 8000 and most preferably about about 4000. These are used at levels of from about 0.20% to 5% more preferably from about 0.25% to 2.5% by weight. These polymers and the previously mentioned homo- or co-polymeric poly-carboxylate salts are valuable for improving whiteness maintenance, fabric ash deposition, and cleaning performance on clay, proteinaceous and oxidizable soils in the presence of transition metal impurities.

Soil release agents are also contemplated for use in the proposed detergenet and cleaning compositions. These can include conventional copolymers or terpolymers of terephthalic acid with ethylene glycol and/or propylene glycol units in various arrangements. Examples of such polymers are disclosed in *e.g.,* U.S. Patent Nos. 4,116,885 and 4,711,730 and EP 0 272 033. Also very useful are modified polyesters as random copolymers of dimethyl terephthalate, dimethyl sulfoisophthalate, ethylene glycol and 1,2-propanediol, the end groups consisting primarily of sulphobenzoate and secondarily of monoesters of ethylene glycol and/or 1,2-propanediol. The target is to obtain a polymer capped at both end by sulphobenzoate groups, "primarily", in the present context most of said copolymers herein will be endcapped by sulphobenzoate groups. However, some copolymers will be less than fully capped, and therefore their end groups may consist of monoester of ethylene glycol and/or 1,2-propanediol, thereof consist "secondarily" of such species.

Also contemplated are the use of softening agents in detergent compositions. These agents may be inorganic or organic in type. Inorganic softening agents are exemplified by the smectite clays as disclosed for example in GB-A-1 400898 and in U.S. Patent No. 5,019,292. Organic fabric softening agents include the water insoluble tertiary amines as disclosed in GB-A1 514 276 and EP 0 011 340 and their combination with mono C₁₂-C₁₄ quaternary ammonium salts are disclosed in EP-B-0 026 528 and di-long-chain amides as disclosed in EP 0 242 919. Other useful organic ingredients of fabric softening systems include high molecular weight polyethylene oxide materials as disclosed in EP 0 299 575 and 0 313 146.

Levels of smectite clay are normally in the range from about 5% to 15%, more preferably from about 8% to 12% by weight, with the material being added as a dry mixed component to the remainder of the formulation. Organic fabric softening agents such as the water-insoluble tertiary amines or di-long chain amide materials are incorporated at levels of from about 0.5% to 5% by weight, normally from about 1% to 3% by weight whilst the high molecular weight polyethylene oxide materials and the water soluble cationic materials are added at levels of from about 0.1% to 2%, normally from about 0.15% to 1.5% by weight. These materials are normally added to the spray dried portion of the composition, although in some instances it may be more convenient to add them as a dry mixed particulate, or spray them as molten liquid on to other solid components of the composition.

Another component for use in detergent compositions are polymeric dye-transfer inhibiting agents. These agents can be included in the detergent compositions from about 0.001% to 10%, preferably from about 0.01% to 2%, more preferably form about 0.05% to 1% by weight of polymeric dye-transfer inhibiting agents. Said polymeric dye-transfer inhibiting agents are normally incorporated into detergent compositions in order to inhibit the transfer of dyes from colored fabrics onto fabrics washed therewith. These polymers have the ability of complexing or adsorbing the fugitive dyes washed out of dyed fabrics before the dyes have the opportunity to become attached to other articles in the wash. Especially suitable polymeric dye-transfer inhibiting agents are polyamine N-oxide polymers, copolymers of N-vinyl-pyrrolidone and N-vinylimidazole, polyvinylpyrrolidone polymers, polyvinyloxazolidones and polyvinylimidazoles or mixtures thereof. Addition of such polymers also enhances the performance of the enzymes according the invention.

It will be apparent to those skilled in the art that various modifications and variation can be made in the compositions, compounds and methods described herein.

### EXAMPLES

### Example 1

### Synthesis and Purification of Enzyme

The pectate lyase from a derivative of *Bacillus subtilis* subsp. *subtilis* str. 168 was produced in *B. subtilis* using the *aprE* promoter for efficient transcription of the gene and the AprE signal sequence for the efficient secretion of the pectate lyase into the culture medium. The *pel* gene was amplified from chromosomal DNA (isolated from a derivative of *Bacillus subtilis* subsp. *subtilis* str. 168 by standard phenol extraction techniques) by PCR using the forward oligonucleotide primer, 5'- TTCAGCGAAGACAGCGCGCAGGCAGCTGATTTAGG CCACCAGACGTTGGG-3' (SEQ ID NO: 3) and the reverse primer, 5'-TTTTCAAAGCTTTA ATTTAATTTACCCGCACCCGCTTGATTT-3' (SEQ ID NO: 4). The forward primer fuses the coding sequence of the AprE signal sequence in frame to the coding sequence of the mature *pel* gene. The forward primer also includes a *Bbs*I restriction site that after cleavage generates an overhang sequence that is compatible with a *Bss*HII site. The reverse primer introduces a *Hind*III restriction site after the stop codon of the *pel* gene coding region. The PCR reaction was performed using Herculase® polymerase (Stratagene, La Jolla, CA) essentially as recommended by the manufacturer. The approximately 1.2 kilobase (kb) PCR fragment was purified (PCR Purification Kit, Qiagen Inc., Valencia, CA) and digested with *Bbs*I and *Hind*III. The expression vector, a derivative of p2JM103, was digested with *Bss*HII and *Hind*III, the vector band isolated and then ligated to the digested PCR fragment carrying the *pel* gene. The resulting expression vector, p2JM103peI, has approximately 700 bp of the *aprE* promoter and signal sequence (*see*, Ferrari et al., 170 J. BACT. 170: 289-295 (1988); and Henner et al., 170 J. BACT. 170: 296-300 (1988)), starting at the *Eco*RI site upstream of the *aprE* promoter and ending at a *Bss*HII site that had been introduced in the AprE signal sequence. The *Eco*RI site of the *aprE* DNA fragment had been cloned into the *Eco*RI site of the pJM103 integrational plasmid (Perego, "Integrational vectors for genetic manipulation in Bacillus subtilis," in BACILLUS SUBTILIS AND OTHER GRAM-POSITIVE BACTERIA: BIOCHEMISTRY, PHYSIOLOGY, AND MOLECULAR GENETICS 615-624 (Sonenshein, Hoch, and Losick (eds), American Society for Microbiology, Washington D.C., 1993). The DNA sequence of the *aprE* fragment from the *Eco*RI to *Bss*HII restriction sites is: 5'-GAATTCTCCATTTTCTTCTGCTATCAAAATAACAGACTCGTGATTTTCCAAACGAG CTTTCAAAAAAGCCTCTGCCCCTTGCAAATCGGATGCCTGTCTATAAAATTCCCGAT ATTGGTTAAACAGCGGCGCAATGGCGGCCGCATCTGATGTCTTTGCTTGGCGAATGT TCATCTTATTTCTTCCTCCCTCTCAATAATTTTTTCATTCTATCCCTTTTCTGTAAAGT TTATTTTTCAGAATACTTTTATCATCATGCTTTGAAAAAATATCACGATAATATCCAT TGTTCTCACGGAAGCACACGCAGGTCATTTGAACGAATTTTTTCGACAGGAATTTGC CGGGACTCAGGAGCATTTAACCTAAAAAAGCATGACATTTCAGCATAATGAACATT TACTCATGTCTATTTTCGTTCTTTTCTGTATGAAAATAGTTATTTCGAGTCTCTACGG AAATAGCGAGAGATGATATACCTAAATAGAGATAAAATCATCTCAAAAAAATGGGT CTACTAAAATATTATTCCATCTATTACAATAAATTCACAGAATAGTCTTTTAAGTAA GTCTACTCTGAATTTTTTTAAAAGGAGAGGGTAAAGAGTGAGAAGCAAAAAATTGT GGATCAGCTTGTTGTTTGCGTTAACGTTAATCTTTACGATGGCGTTCAGCAACATGA GCGCGC-3' (SEQ ID NO:5). The 3' end of the *pel* gene was cloned upstream of the LAT terminator (from the *Bacillus licheniformis* α-amylase gene, Yuuki et al., 98 J. BIOCHEM (TOKYO) 1147-56 (1985)) using a *Hind*III site that had been introduced at the 5' end of the terminator. The 3' end of the LAT terminator had been cloned into the *Hind*III site of the pJM103 plasmid as an a oligonucleotide so that the original *Hind*III site in the plasmid was eliminated after ligation. The DNA sequence of the LAT terminator region from the *Hind*III site to the dead *Hind*III site in pJM103 is:
5'-AAGCTTAACTCGAGGTTAACAGAGGACGGATTTCCTGAAGGAAATCCGTTTTTTT ATTTTTAATTAAGAGCTT-3' (SEQ ID NO: 6).

To construct the production host, pJM103pel was used to transform *B. subtilis* BG3594comK *(degU^{Hy}32, oppA,* Δ*spoIIE,* Δ*aprE,* Δ*nprE, amyE::xylRPxylAcomK-phleo).* Competent cells were made by the induction with xylose of the *comK* gene driven by the *xylA* promoter (Hahn et al., 21 MOL. MICROBIOL. 763-75 (1996)). Transformants were selected on Luria Broth agar plates with 5 µg/mL chloramphenicol and selected colonies were serially streaked on Luria Broth agar plates with 25 µg/mL chloramphenicol until rapid colony growth was obtained in order to select for clones with higher gene copy numbers. For the production of Pel, cutures were grown in 14 L fermentors

Pectate lyase production is based in part on *Bacillus subtilis* fermentation, recovery and formulations known in the art, and can be adjusted accordingly.

To grow an in inoculum, Luria-Bertoni (LB) broth was supplemented with 1% glucose and 25 ppm chloramphenicol antibiotic (used as a strain marker) was added to the broth. Similar nutrient-rich medias can be substituted for LB broth. The medium was seeded with cells that had been frozen in a glycerol stock in a bath of liquid nitrogen. The seed was grown in 2 L Erlenmeyer flasks containing 600 mL of the prepared medium, shaken at 200 rpm at 37°C. The cultured inoculum was then transferred to a fermentor when the optical density (OD) at 550 nm was about 1. The fermentor is batched with medium components at the concentrations provided below based on 0+ (batch size after inoculum transfer).

| **Raw Material** | **Concentration (g/kg 0+)** |
|---|---|
| Soy flour | 140.0 |
| KH₂PO₄ | 5.4 |
| NaH₂PO₄ aq. | 10.7 |
| Citric Acid | 0.13 |
| FeCl₃·6aq | 0.09 |
| MnCl₂·4aq | 0.06 |
| MgSO₄·7aq | 4.0 |
| Antifoam | 2.0 |
| Cellulase, hemicellulase, pectin enzymes | 2.0 |
| Glucose | 5.0 |

Fermentation medium was treated with industrial blends of cellulases, hemicellulases and pectinases, as indicated in the above table, to make the solution less viscous. To feed the batch, 600 g glucose per kg of sterilized solution was used. Limited glucose and excess dissolved oxygen (DO) levels were maintained until harvest. Ammonia solution was used to control the pH at 7.4. The fermentor was cooled to maintain the temperature at 37°C. Total fermentation time was 36 hours.

After harvest, the fermentation broth was cooled. The broth was then treated with salts and flocculants and filtered to remove cells. The material was then ultrafiltered to concentrate the pectate lyase. The concentrated pectate lyase was formulated to pH 6.25 with the stabilizers of 40% propylene glycol and 0.25% Proxel.

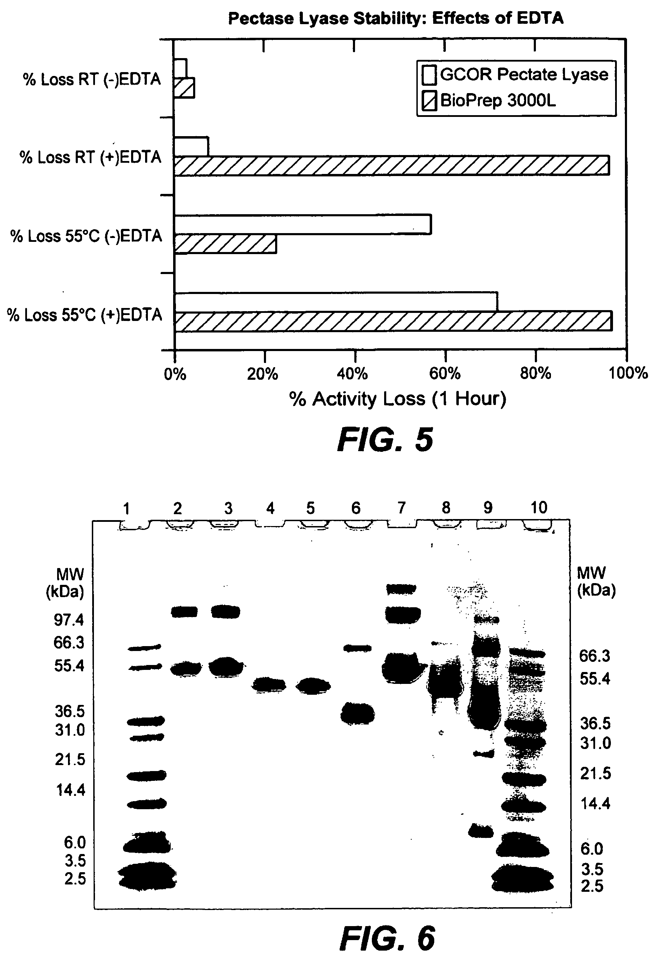

Concentrated pectate lyase was run on a NuPAGE 4-12% Bis-Tris gel with MES running buffer. A Mark12 standard molecular weight (MW) marker was run with the samples (lanes 1 and 10). Fig. 6 shows the gel. Lane 2 has 2.5 µg total protein of bovine serum albumin (BSA)(control). Lane 3 has 5 µg BSA. Lane 4 has concentrated pectate lyase obtained as described above at a concentration of 5 µg per lane and as diluted with HCl. Lane 5 has concentrated pectate lyase obtained as described above at a concentration of 5 µg per lane and as diluted with deionized (DI) water. Lane 6 has 5 µg of the Novozymes Bioprep 3000L. Lane 7 has 10 µg of BSA. Lane 8 has 10 µg of the pectate lyase obtained as described above and as diluted with HCl. Lane 9 has 10 µg of the Novozymes Bioprep 3000L.

### Example 2

### Characterization of Pectate Lyase

Pectin removal performances of the *B. subtilis* pectate lyase (SEQ ID NO: 1) were compared to the commercially available pectate lyase, Bioprep 3000L (Novozymes), under the following conditions.

| Pectate Lyases | Total Protein* mg/mL | TCA Protein* (mg/mL) |
|---|---|---|
| Exp. *B. subtilis* pectate lyase of | 87.8 | 53.4 |
| Example 1 | | |
| Bioprep 3000L (Novozymes) | 44.0 | 35.5 |

| | | |
|---|---|---|
| "*" The results are calculated based on a conversion of 6.35 g protein/g nitrogen. Additionally the results are a total protein estimation. | | |

***Materials.*** The substrate utilized was Army Carded Cotton Sateen, desized but not bleached fabric from Testfabrics (Style #428). *B. subtilis* pectate lyase from Example 1 arid Novozymes Bioprep 3000L pectate lyase were utilized at 2 ppm total protein. 50 mM BIS-TRIS propane (BTP) buffer at various pH's were analyzed. The buffer was adjusted using HCl. 0.005% Ruthenium Red dye in a 50 mM phosphate buffer solution (pH 6.0) was used.

***Procedures.*** To perform the pH and temperature profiles, the following was performed. Preliminary pH and temperature profiles of pectin removal by the enzymes were obtained by incubating 1.6 cm (5/8 inch) pieces of cotton fabric (Testfabrics style #428) disks in a 12-well micro-titer plate. 2 mL total final volume of BTP/HCl buffer and enzyme were added into each well of 12-well microtiter plate along with one fabric disk. After sealing the plate tightly with a plate sealer, the 12-well plate was placed into the incubator shaker and incubated for 60 minutes under target temperature at 250 rpm. The discs were thoroughly rinsed with deionized water. After rinsing, 1.5 mLs of 0.05% Ruthenium Red dye in 50 mM sodium phosphate buffer at pH 6 was placed in each well containing a fabric disc. The plate was then incubated again in the incubator shaker for 15 minutes at 250 rpm. The fabric discs were then taken out of the Ruthenium Red dye solution and rinsed with deionized water and air-dried.

To quantify pectin removal by the enzymes, CIE L* values of each stained fabric disc were measured using a spectrophotometer (Minolta CR-200).

The *B. subtilis* pectate lyase of Example 1 and Bioprep 3000L were also tested for their dose response activity in removing pectin using a Launder-Ometer. To check pectin removal efficacy, three 10.2 cm by 7.6 cm (4 inch by 3 inch) swatches of Army Carded Cotton Sateen fabric (Testfabrics style #428, desized but not bleached) were treated in a Launder-Ometer at 55°C for 20 minutes at pH 7.5 in 50 mM BTH/HCl buffer for the pectate lyase of Example. Bioprep 3000L was tests with equivalent swatches in 50 mM BTH/HCl at a pH 8.2.

After incubation, all the fabric swatches were thoroughly rinsed with water and then air dried before Ruthenium Red staining. Three 1.6 cm (5/8 inch) fabric discs were cut from each of the treated fabric swatches. These discs were then stained with 0.05% Ruthenium Red dye. Dyeing was performed in 50 mM sodium phosphate solution, at pH 6. Pectin removal of each treatment was quantified according to the procedures described above for the pH and temperature profiles.

***Results.*** Pectin removal over preliminary pH and temperature profiles are provided in FIG. 2. Pectin removal was measured by Ruthenium Red dye staining. Pectin removal performance using the *B. subtilis* pectate lyase demonstrated a broad temperature profile and optimal pH range of about 6 to 8 as shown in Fig. 1. The lower CIE *L value indicates higher pectin binding. The higher the pectin binding indicates a lower pectin removal performance by the enzyme. Other scouring assays can be used, such as that described in Arne Solbak, et al., "Discovery of pectin-degrading enzymes and directed evolution of a novel pectate lyase for processing cotton fabric," 280 J. BIOL. CHEM. 9431-9438 (2005).

The pectate lyase of Example 1 was also tested for its dose response of pectin removal using a Launder-Ometer. The results are displayed in FIG. 3.

To compare pectin removal efficacies of the *B. subtilis* pectate lyase and Novozymes Bioprep 3000L pectate lyase, experiments were performed as follows. Three pieces of 10.2 cm by 7.6 cm (4 inches by 3 inches) desized Army carded cotton sateen fabric (Testfabrics, style #428) were treated with varied concentrations of pectate lyase at 55°C for 20 minutes in a Launder-Ometer. To compare the pectate lyases under its optimal pH, the pH of 7.5 was used for the GCOR pectate lyase and a pH of 8.2 was used for the Bioprep 3000L. After the 20 minute treatment, the fabric swatches were thoroughly rinsed with deionized water and air-dried. The 1.6 cm (5/8 inch) disks were cut from each fabric swatch, and the fabric disks were stained with 0.05% Ruthenium Red dye to quantify pectin removal as described previously.

The pectate lyase of Example 1 was also assessed for peroxide stability at 25°C and 55°C. Pectate lyase activity increased at the lower temperature of 25°C over its activity at the higher temperature and over the activity of Bioprep 3000L. Pectate lyases activity was measured using 0.2% (w/v) polygalacturonic acid in 25 mM Tris/HCl, 25 mM glycine/NaOH, pH 9, buffer. Activity was determined by measuring a linear increase in absorbance at 235 nm for 5 minutes using a spectrophotometer. One unit of enzyme activity was defined as the amount of protein that produced 1 µmol unsaturated oligogalacturonides/minute, which is equivalent to 1 µmol unsaturated digalacturonide, using a molecular extinction coefficient for the dimer of 4600 M⁻¹ cm⁻¹ at 235 nm. Protein concentration was determined by measuring absorbance at 280 nm, using an extinction coefficient based on the amino acid sequence of the pectate lyase of Example 1. Specific activity was determined as units/mg of purified pectate lyase. The pectate lyase activity assay is as described in Arne Solbak, et al., "Discovery of pectin-degrading enzymes and directed evolution of a novel pectate lyase for processing cotton fabric," 280 J. BIOL. CHEM. 9431-9438 (2005). Enzyme stability in the presence of increasing amounts of peroxide were assessed using the pectate lyase activity assay as described. The results of the test are displayed in Figs. 4A and 4B.

The pectate lyase of Example 1 was also tested for its enzymatic stability in the presence of the chelator, EDTA, using the pectate lyase activity assay as described by Solbak et al. (2005). Washing compositions preferably lack divalent cations such as calcium. However, the presence of cations is often required for optimal enzymatic activity. Fig. 5 shows that there was little loss of enzymatic activity of the pectate lyase from Example 1 when compared between samples with and without EDTA at room temperature. However, there was significant loss of function of the enzyme when the temperature was taken to 55°C as compared to the other enzymes tested.

Based on the results, another aspect contemplates taking the purified pectate lyase from. Example 1 and treating it with a peroxide, such as hydrogen peroxide during recovery to produce a pectate lyase product with significantly higher activity than the untreated pectate lyase. For example, with treatment of hydrogen peroxide the pectate lyase may obtain a 10% gain in specific activity. The treated pectate can then be used in the various compositions and one-step processes described in the application.

### Example 3

The pectate lyase from Example 1 was formulated with propylene glycol (40%), Proxel (0.25%) (1,2-benzisothiazolin-3-one), and at a pH of 6.25±0.1 using acetic acid. The assay was performed at 37°C for a 20 minute incubation. The end point measurement was performed using a glycine buffer with CaCl₂ and a substrate of polygalacturanic acid.

| Ferm. Pectate Lyase | Assay vol. (µL) | OD@235 nm at pH 10 | Blind 210 | Final OD@235 nm pH 10 | APSU at pH 10 | APSU/mL of sample |
|---|---|---|---|---|---|---|
| 20040772 UFC | 500 | 0.712 | 0.124 | 0.588 | 0.137458362 | 91638.91 |
| 20040773 UFC | 500 | 0.613 | 0.124 | 0.489 | 0.116094893 | 77396.6 |
| 20040772 Final | 500 | 0.336 | 0.124 | 0.212 | 0.056320337 | 37546.89 |
| 20040773 Final | 500 | 0.467 | 0.124 | 0.343 | 0.08458917 | 56392.78 |
| 20040396 whole broth | 500 | 0.415 | 0.124 | 0.291 | 0.073367954 | 24455.98 |
| 20040397 whole broth | 500 | 0.3055 | 0.124 | 0.1815 | 0.049738662 | 16579.55 |

The final APSU/mL is calculated using the following formula:

APSU/mL in sample = APSU/mL from standard curve x K x F/a, wherein K is the dilution factor by which the sample is diluted; F is 2, as 1:1 mixture of enzyme and substrate mixture is used; a is the pipetted volume of enzyme sample prior to dilution in mL. Once the density of the samples is known, the units of APSU/mL can be converted to APSU/g. These calculations are based upon the Bioprep 3000L sample having 3000 APSU/g and a standard curve was generated from that sample. The standard curve from the Bioprep 300L sample had a slope of 0.215793, an intercept of 0.010572 and a RSQ of 0.9982. If the activity is lost during long storage of the enzyme, say 10-fold, such that the Bioprep 3000L has only 300 APSU/g, then all the activity numbers in the samples containing the pectate lyase from *B. subtilis* will also decrease 10-fold. This end-point assay requires a standard with known activity to measure activity of unknown samples. decrease 10-fold. This end-point assay requires a standard with known activity to measure activity of unknown samples.

### Example 4

### Cleaning Compositions comprising Pectate Lyase and Activity

*Bacillus subtilis* pectate lyase exhibited cleaning of various carbohydrate containing soils in North American laundry conditions. The pectate lyase of Example 1 was tested in AATCC standard heavy-duty liquid detergent (HDL) and Dial's commercial Purex liquid detergent in full-scale top-loader washing machines and small-scale Tergotometer at sub-parts per million concentrations. Cleaning performance of the pectate lyase was compared against amylases from *Bacillus licheniformis* and *Bacillus* sp. 707.

Tests were performed in two machine scales, a Tergotometer (United States Testing Co. Inc., Hoboken, NJ, USA) and a top-loading US washing machine (Kenmore Elite model 110.26962503, Sears Roebuck and Co., Hoffman Estates, IL, USA). Stained swatches were obtained from the Center for Testmaterials BV (CFT, Vlaardingen, Netherlands), EMPA Testmaterials AG (St. Gallen-Winkeln, Switzerland), and Warwick-Equest (Consett, United Kingdom). Aged technical stains acquired from CFT and EMPA were CFT CS-2 Cocoa, CFT CS-20 Tomato, CFT CS-6 Salad Dressing with Natural Black, EMPA160 Chocolate Ice Cream, EMPA 161 Starch on cotton, EMPA163 Porridge, and EMPA164 Grass. Fresh stained fabric tested from Warwick-Equest were scrubbed grass and 4 x 4 multi-stain panels consisting of various carbohydrate containing stains including Mixed Berries, Tomato Soup, Ragu Traditional Pasta Sauce, Curry Blend, C&G Cheese and Broccoli Baby Food, Heinz Vegetable and Turkey Sauce, Coleman's Mustard, Coleman's Gravy, Bisto Gravy, Kellogg's Cocoa Pops, Fresh Banana, Black Olives, HP BBQ Sauce, HP Brown Sauce, Homepride Chilli Con Came, Tomato Ketchup, Strawberry Ice Cream, Nutella Chocolate Spread, Schwartz Paprika, Econa Hot Pepper Sauce, Coleman's Sausage Casserole, Patak's Doplaza, Patak's Vindaloo, Uncle Ben's Madras, Frijj Chocolate Milkshake, Pec Bilberries, JW Blackcurrant Juice, Robertson's Blackberry Jam, Heinz Apple and Banana baby food, Coleman's Beef Casserole, Heinz Chocolate Pudding Baby Food, Cadbury's Chocolate Mousse, St. Dalfour's Blueberry Marmalade, Morton's Red Cherry Pie Filling, Fresh Plums, Asda Prunes, Asda Curry Ketchup, Apple, Orange, Banana, Rusk Baby Food, Heinz Sun Dried Tomato Sauce, Frijj Strawberry Milkshake, V8 Vegetable Juice, Tea, Coffee, Merlot Red Wine, Baxter's Beetroot, Welch's Purple Grape Juice, J. West Black Berry, Napolina Chopped Tomato, Homepride Tikka, and QuinkBlue Ink. Each stain was measured before and after treatment by optical reflectance using a Minolta Reflectometer CR-400 for the multi-stain and CR-410 for the technical soils. The difference in the L, a, b values was converted to total color difference (dE), as defined by the CIE-LAB color space. Cleaning of the stains are expressed as percent stain removal index (%SRI) by taking a ratio where the color difference between the initial stain and cleaned stain is divided by the color difference between the initial stain and the unsoiled fabric. Typically 9 replicates were tested in tergotometer scale, triplicate swatches were run in triplicate pots, while 8 replicates were tested in full-scale, quadruplicate swatches in duplicate machines.

Small scale cleaning experiments were conducted in a Tergotometer. Approximately 1 L of Milli-Q reverse osmosis water was added to each pot to which 1.5g/L AATCC HDL WOB 2003 liquid detergent, 5mM HEPES pH 8.0, and 1.6 grains per liter (6 grains per gallon) water hardness (at a 3:1 Ca:Mg ratio) was also added and temperature was allowed to equilibrate to 25°C (77°F). The solution volume was occasionally adjusted to maintain 40g fabric per liter wash liquor when the 3 multi-stain swatches added to each pot exceeded 40g. Enzyme and stains were added immediately before starting the wash, which was agitated at 100 rpm for 12 min at 25°C (77°F). Following cleaning, swatches were rinsed for 3 minutes in tap water, spun to remove excess water and allowed to air dry overnight. Soil removal was assessed by reflectometry as described above and expressed as percent soil removal index (%SRI). Data is also represented in delta %SRI, where the %SRI of the control treatment, detergent alone, is subtracted from the treatments with enzymes.

Full-scale cleaning was conducted in a Kenmore Elite King size washer set to Ultra Clean Cylce (15 min), small load (48L), Cold/Cold Autotemp, one rinse, slow agitation and fast spin. Either 1.5g/L AATCC WOB 2003 heavy-duty liquid detergent and 5mM HEPES pH8.0 or 1.5g/L Purex liquid detergent was added along with 1.6 grains per liter (6 grains per gallon) water hardness (at a 3:1 Ca:Mg ratio) as the washing machine filled. Temperature was controlled to 21°C (70°F) by addition of warm water or ice, as needed, once the machine had finished filling. Enzymes, soiled swatches and ballast were then added to the machine and the cycle was allowed to proceed. After the cleaning cycle was completed swatches were allowed to air dry overnight. Soil removal was assessed by reflectometry and expressed as percent soil removal index (%SRI) and delta %SRI, as described above.

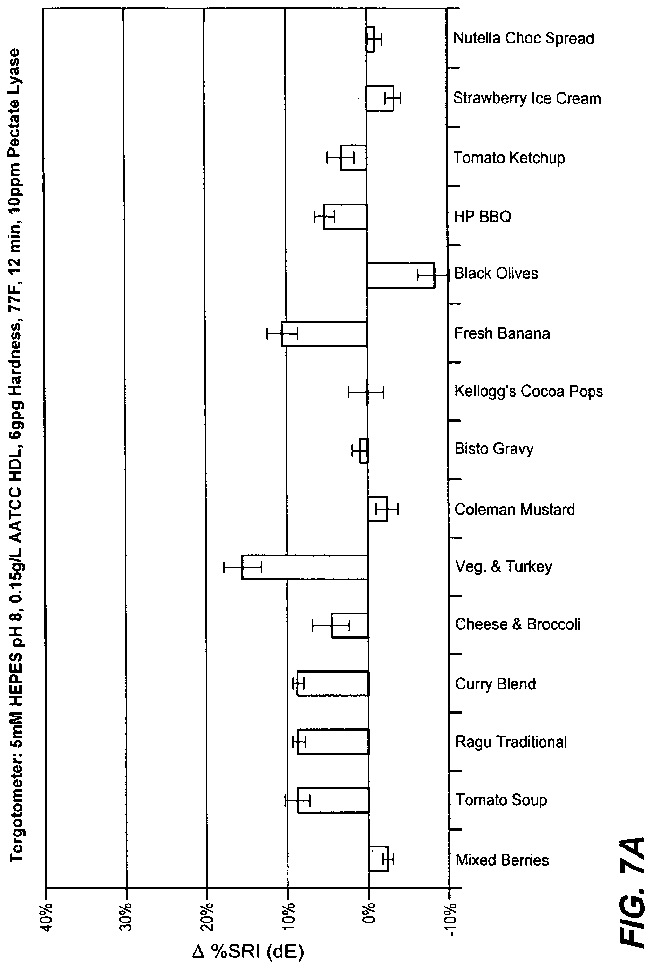

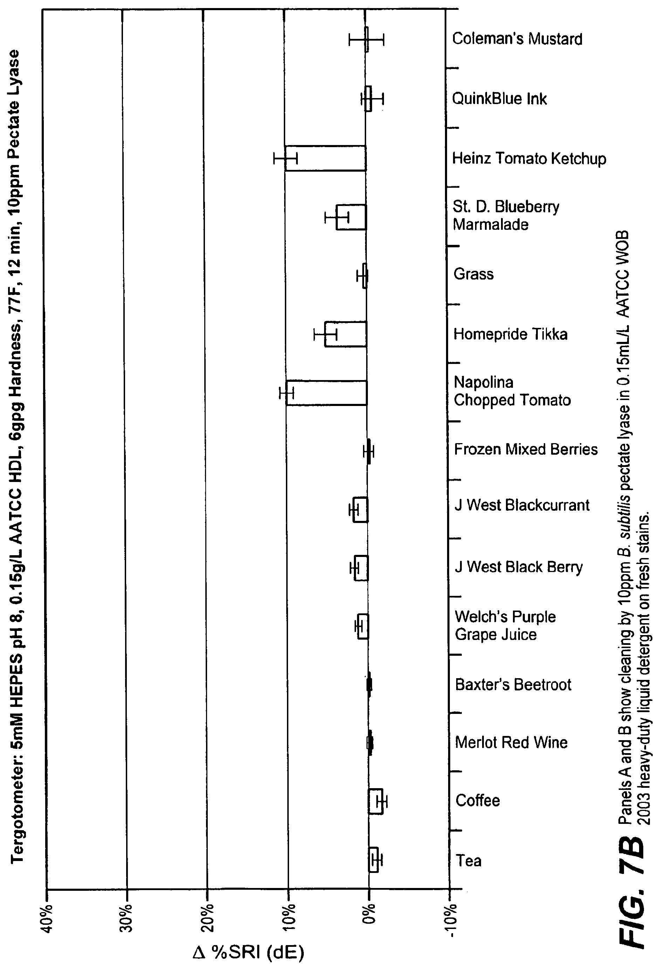

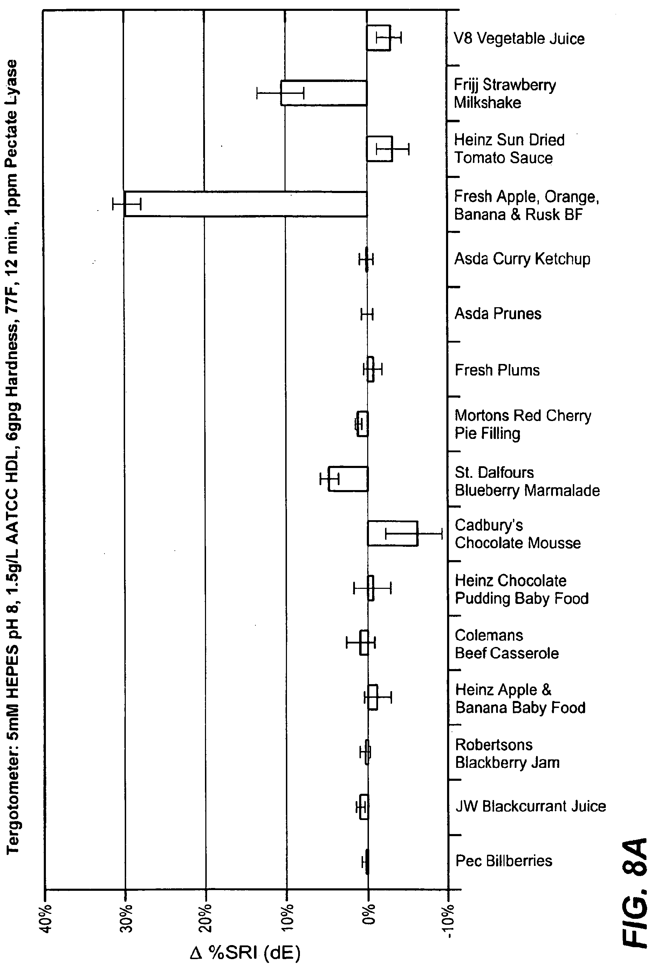

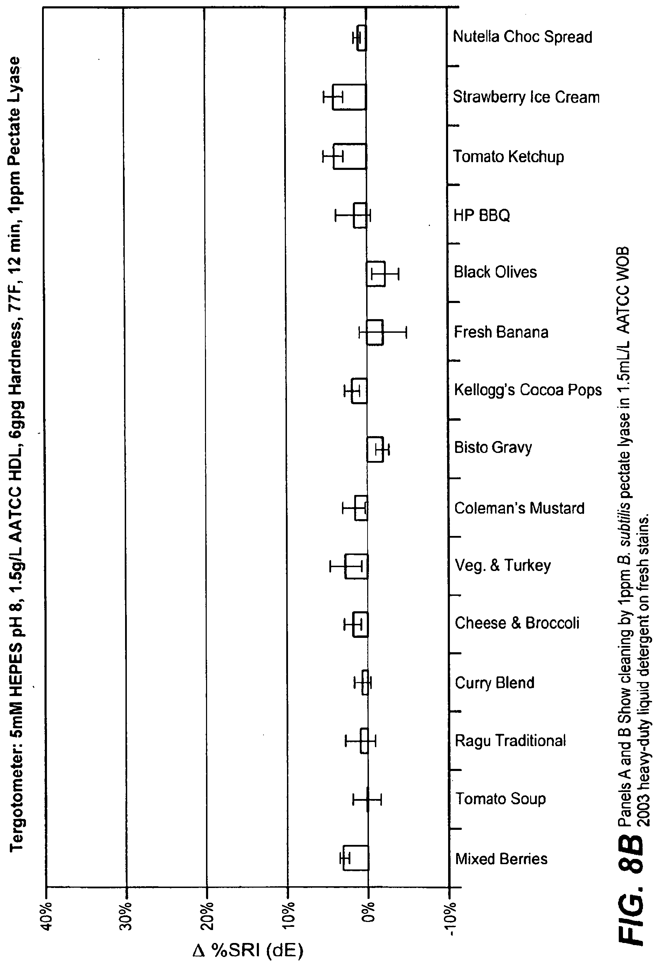

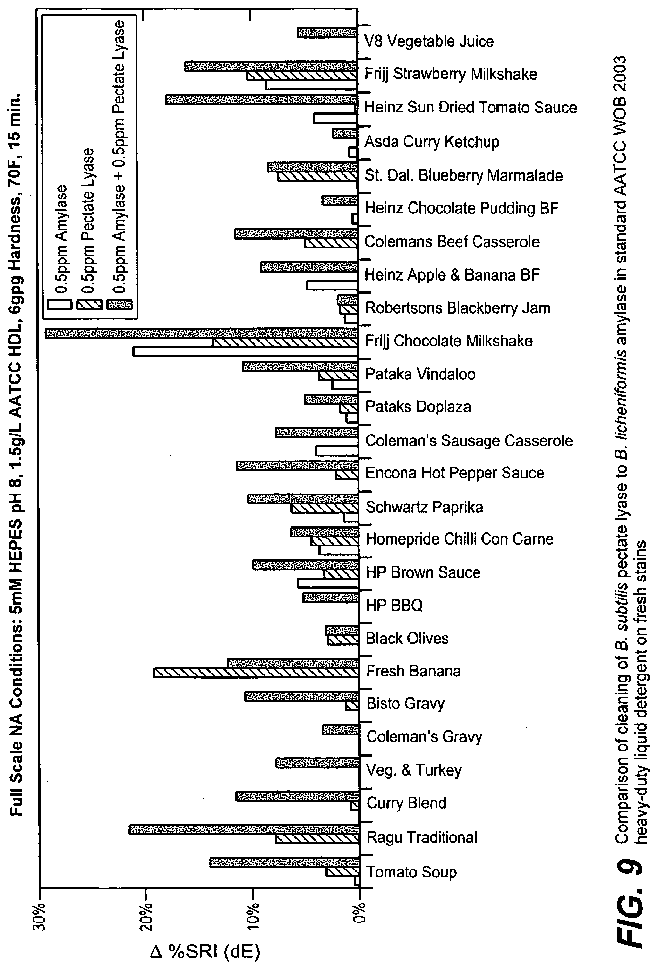

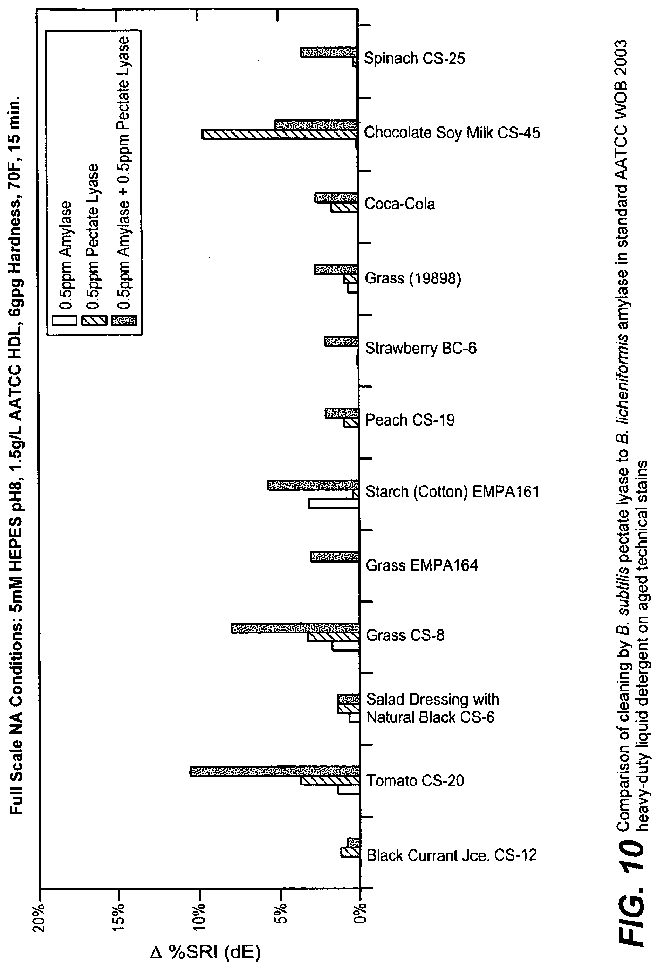

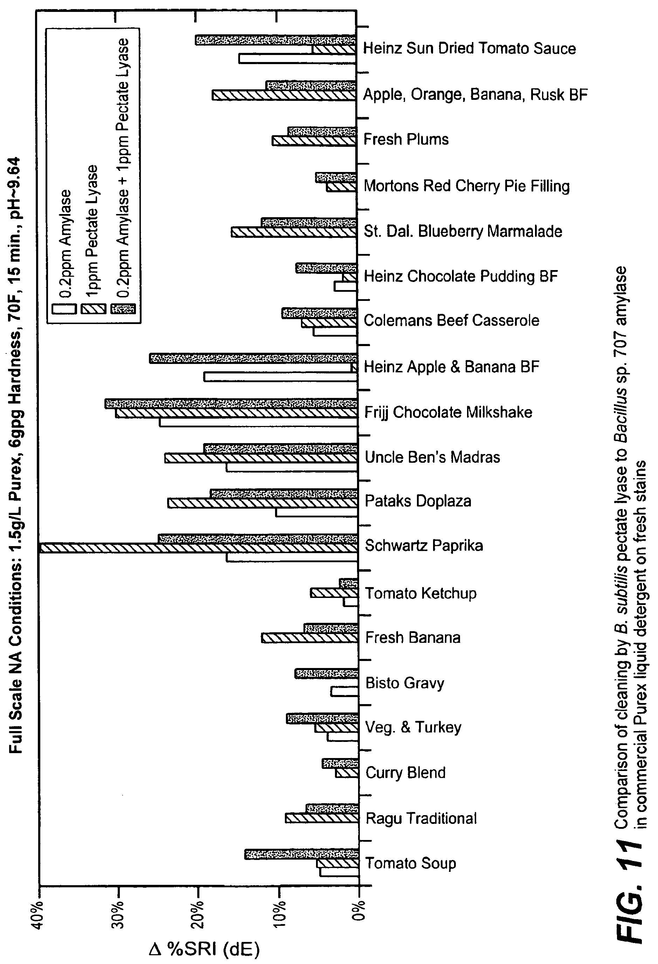

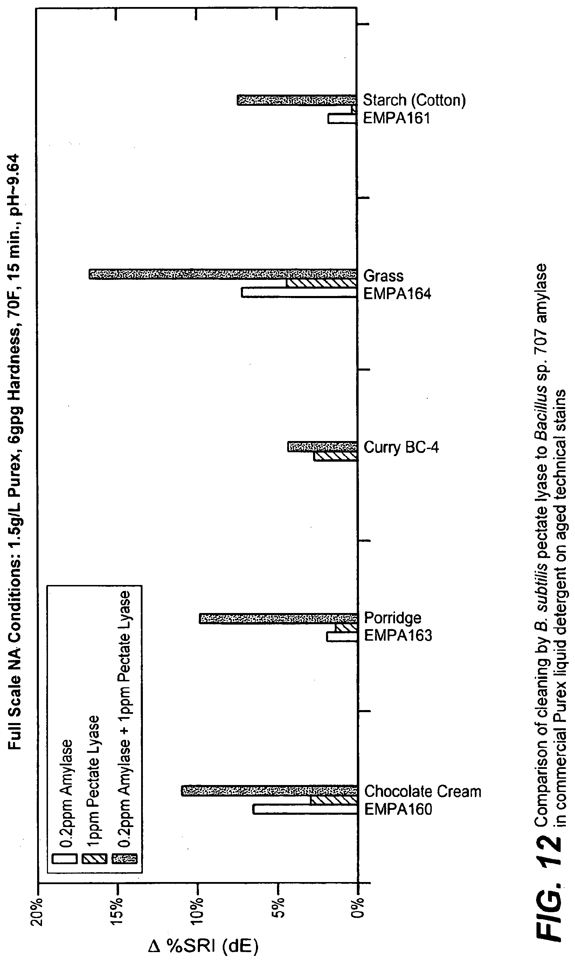

The *Bacillus subtilis* pectate lyase proved effective at cleaning many fresh consumer relevant soils as well as aged technical soils (see Figures 7-12). The contribution to cleaning by pectate lyase is much more pronounced in the AATCC HDL at pH 8.0 than in Purex liquid detergent at pH 9.6. It showed significant cleaning down to 0.5 ppm, showed cleaning on its own, and exhibited synergy with amylase in cleaning some of the stains not attributed to additive effects of combining the enzymes.

## Claims

1. A method for scouring and bleaching a textile comprising contacting the textile for a length of time and under conditions suitable to permit whitening of the textile, with an aqueous solution comprising a chemical bleaching agent which is hydrogen peroxide or an enzymatic bleaching system, and a pectate lyase obtained from *Bacillus subtilis* having a molecular weight of about 43 kD, a pI of about 7.3 under reducing SDS-PAGE conditions, and an optimal pH of about 5.0 to 11.0,
wherein the enzymatic bleaching system comprises enzyme, ester substrate, and hydrogen peroxide and produces peracids by enzymatic perhydrolysis.

2. The method of claim 1, wherein the suitable conditions comprise:
(i) a pH which is between about 6 and about 8; or
(ii) a length of time between about 2 minutes and 24 hours, which is optionally between about 15 minutes and 12 hours, which is optionally between about 30 minutes and 6 hours; or
(iii) a temperature of between about 15°C and 95°C, which is optionally between about 20°C and 80°C, which is optionally between about 25°C and 60°C.

3. The method of claim 1, wherein the aqueous solution comprises a chemical bleaching agent which is hydrogen peroxide and which is present in a concentration of between about 100 ppm to 5000 ppm, which is optionally between about 500 ppm to 4000 ppm, which is optionally between about 1000 ppm to 3000 ppm.

## Patentansprüche

1. Verfahren zum Entschweißen und Bleichen eines Textilstoffs, umfassend das Kontaktieren des Textilstoffs für eine Zeitspanne und unter Bedingungen, die geeignet sind, das Bleichen des Textilstoffs zu gestatten, mit einer wässrigen Lösung umfassend ein chemisches Bleichmittel, das Wasserstoffperoxid oder ein enzymatisches Bleichsystem ist, und einer Pectatlyase, die aus Bacillus subtilis erhalten worden ist, und ein Molekulargewicht von etwa 43 dD, einen pI-Wert von 7,3 unter reduzierenden SDS-PAGE-Bedingungen und einen optimalen pH-Wert von etwa 5.0 bis 11,0 aufweist,
wobei das enzymatische Bleichsystem Enzym, Estersubstrat und ein Wasserstoffperoxid umfasst und Persäuren durch enzymatische Perhydrolyse erzeugt.

2. Verfahren nach Anspruch 1, wobei die geeigneten Bedingungen Folgendes umfassen:
(i) einen pH-Wert, der zwischen etwa 6 und etwa 8 liegt; oder
(ii) eine Zeitspanne zwischen etwa 2 Minuten und 24 Stunden, die wahlweise zwischen etwa 15 Minuten und 12 Stunden liegt, die wahlweise zwischen etwa 30 Minuten und 6 Stunden liegt; oder
(iii) eine Temperatur zwischen etwa 15 °C und 95 °C, die wahlweise zwischen etwa 20 °C und 80 °C liegt, die wahlweise zwischen etwa 25 °C und 60 °C liegt.

3. Verfahren nach Anspruch 1, wobei die wässrige Lösung ein chemisches Bleichmittel umfasst, das Wasserstoffperoxid ist und das in einer Konzentration zwischen etwa 100 ppm und 5000 ppm vorliegt, die wahlweise zwischen etwa 500 ppm und 4000 ppm beträgt, die wahlweise zwischen etwa 1000 ppm und 3000 ppm beträgt.

## Revendications

1. Procédé d'abrasion et de blanchiment d'un textile comprenant la mise en contact du textile sur une durée et sous des conditions appropriées pour permettre le blanchissement du textile, avec une solution aqueuse comprenant un agent chimique de blanchiment qui est le peroxyde d'hydrogène ou un système de blanchiment enzymatique, et une pectate lyase obtenue à partir de *Bacillus subtilis* ayant un poids moléculaire d'environ 43 kD, un pI d'environ 7,3 sous des conditions SDS-PAGE réductrices, et un pH optimal d'environ 5,0 à 11,0,
où le système de blanchiment enzymatique comprend une enzyme, un substrat ester, et un peroxyde d'hydrogène et produit des peracides par perhydrolyse enzymatique.

2. Procédé selon la revendication 1, où les conditions appropriées comprennent:
(i) un pH qui est compris entre environ 6 et environ 8; ou
(ii) une durée comprise entre environ 2 minutes et 24 heures, qui est optionnellement comprise entre environ 15 minutes et 12 heures, qui est optionnellement comprise entre environ 30 minutes et 6 heures; ou
(iii) une température comprise entre environ 15°C et 95°C, qui est optionnellement comprise entre environ 20°C et 80°C, qui est optionnellement comprise entre environ 25°C et 60°C.

3. Procédé selon la revendication 1, où la solution aqueuse comprend un agent chimique de blanchiment qui est le peroxyde d'hydrogène et qui est présent en une concentration comprise entre environ 100 ppm à 5 000 ppm, qui est optionnellement comprise entre environ 500 ppm à 4 000 ppm, qui est optionnellement comprise entre environ 1 000 ppm à 3 000 ppm.
